(19) Europäisches Patentamt
European Patent Office
Office européen des brevets

(11) EP 1 199 056 A1

(12) EUROPEAN PATENT APPLICATION

(43) Date of publication:
**24.04.2002 Bulletin 2002/17**

(51) Int Cl.[7]: **A61F 13/14**, A61F 13/514

(21) Application number: **00122215.7**

(22) Date of filing: **16.10.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **The Procter & Gamble Company**
**Cincinnati, Ohio 45202 (US)**

(72) Inventors:
- **Toro, Carlo**
  **65012 Cepagatti (Pescara) (IT)**
- **Carlucci, Giovanni**
  **66100 Chieti (IT)**
- **Elliott, Russell Phillip**
  **Egham, Surrey TW20 9RJ (GB)**
- **Duke, Roland Philip**
  **Wokingham, Berkshire RG41 3NB (GB)**
- **Pesce, Antonella**
  **65120 Pescara (IT)**

(74) Representative: **Kremer, Véronique et al**
**Procter & Gamble Service GmbH**
**65824 Schwalbach am Taunus (DE)**

(54) **Breast pads**

(57) The present invention relates to disposable thin breathable breast pads having a wearer facing surface and a garment facing surface, said breast pad having a water vapour permeability of more than 100 (g)/($m^2$/24hrs) and a stiffness of less than 600 grams. These breast pads are able to closely conform to the various anatomical shapes of the breasts of women while offering enhanced comfort and low degree of wearing awareness.
The breast pad has a water vapor permeable backsheet comprising a film and a support layer, and having anti-slip properties on the garment facing surface.

Figure 2

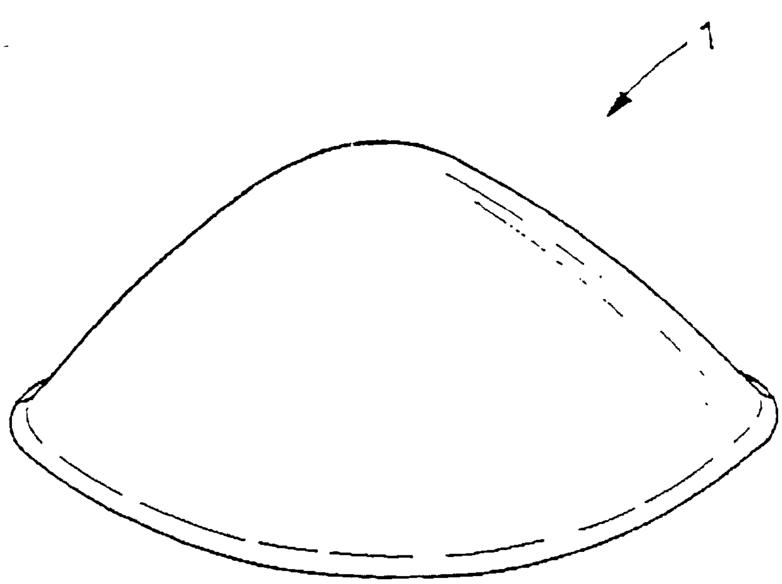


EP 1 199 056 A1

## Description

### Field of the Invention

This invention relates to breast pads suitable to be worn over the breast of nursing mothers and more particularly to thin breast pads offering enhanced fit and comfort during use.

### Background of the Invention

In the recent years women have become increasingly active outside the home and in various work environments. Maternity leaves from employment following the birth of a child are commonly quite short. At the same time, the popularity of breast feeding babies has experienced a resurgence. In order to prevent staining of clothing and embarrassment caused by leaking of fluid, nursing mothers employ pads to absorb leaking fluid. Such pads are commonly placed inside a bra.

Breast pads commercially available do not fulfil all the needs of the nursing women. Some of them are thick and do not comply with the need of discretion during use. Currently available pads are substantially dished or cup-shaped in an attempt to follow the contours of the human breast, however, they are not truly conformable and lack in comfort to the nursing mother. Indeed forming measures may result in a harsh, stiff edge around the pad that can cause chafing or other irritation. Yet another problem is that breast pads have the tendency to disintegrate in wet conditions while being used. Also some of them are not able to efficiently absorb the milk discharge resulting in varying degrees of leaking which frequently causes discomfort and may result in garment staining.

Actually it exists a real consumer need for a breast pad which will closely conform to the various anatomical shapes of the breasts of women while offering enhanced comfort and low degree of wearing awareness. More particularly, it is an object of the present invention to provide breast pads offering enhanced fit and discretion in use while providing enhanced comfort, this without compromising on protection, thereby allaying consumer fears of leakage and staining. It is apparent that such a pad will also need to satisfy the conditions of usefulness in the context of manufacturing a commodity mass product such as high-speed making (at a linear production speed of at least 0.5 km/hour), low cost material availability and environmental friendly availability.

It has now been found that the above needs can be addressed by providing a thin breathable breast pad having a water vapour permeability (as measured by the water vapour permeability test method described herein after) higher than 100 (g) / ($m^2$/24hrs) and a stiffness of less than 600 grams (as measured by the modified ASTM method D4032-94 described herein after). Such a breast pad with enhanced conformability ensures excellent fit to the breast of a woman even when undergoing stress conditions (e.g., during sport or when the mother carries her baby in front of her breasts), while alleviating the occurrence of occlusive environment on the surface of the breast, that might result in discomfort to the woman. The breathability of the pad will allow perspiration liquid or absorbed liquid to dissipate by evaporation, by providing a water vapor exchange through the pad itself (rather than between the pad and the woman's skin). The reduction in the hot, humid and occlusive environment between the vicinity of the skin of the woman and the breast pad itself also reduces the tendency of the woman to perspire. The breathability of the pad according to the present invention allows for the evaporation of fluid/liquid from the pad, and also for a reduction in the amount of perspiration generated by the woman wearing such a breast pad and thus a reduction in hot and sweaty feelings otherwise associated with the presence of non-breathable pad on the skin. The breathable pad according to the present invention therefore needs to retain less liquid and can do so more effectively. The use of such breathable pad typically having breathable backsheet further contributes to a clean and dry facing surface (topsheet), such that the surface feels dry to the touch and the skin of the woman does not feel wet or moist and such that the woman wearing the breast pad experiences minimal discomfort during wearing.

Typically the breast pads according to the present invention deliver the above mentioned benefits while ensuring effective protection. Indeed preferably the breast pads according to the present invention have a fluid absorption capacity of at least 0.05 grams per square centimetre of the pad.

Preferably the breast pads according to the present invention advantageously further maintain their integrity once wetted and hence do not disintegrate during use. This benefit is typically achieved by the use of an absorbent core within the breast pad which has a high tensile strength under use condition (i.e., wet condition).

In a preferred embodiment the breast pads are further provided with non-slip properties on their wearer facing surface and/or the garment/bra facing surface. Such non-slip treated surface typically has a coefficient of static friction of at least 1, as measured by ASTM D 1894.The material used to treat the wearer facing surface and/or the garment/bra facing surface of the breast pad, preferably only slightly impairs or does not impair at all the conformability properties and/or breathability properties of the pads. Typically non-slipping properties are desired on the garment/bra facing surface of the breast pads for improved stay in place.

The pads according to the present invention may have any shape (flat or 3D), form/geometry and/or dimension. Typically they have a three-dimensional (3D) shape so as to better fit to the morphology of women breasts. Any circular form/geometry is suitable for use herein. In a preferred execution the pad has a geometry so that at least more than 50% of the surface area of the pad is disposed beneath the breast nipple. Improved discretion and relative better absorption capacity are achieved.

## Summary of the Invention

The present invention relates to a disposable thin breathable breast pad having a water vapour permeability of more than 100 (g)/(m2/24hrs) and a stiffness of less than 600 grams. Preferably the pad has a fluid absorption capacity of at least 0.05 grams per square cm of the pad.

It is generally preferred that the breast pad of the invention comprises an absorbent core disposed between a liquid and water vapor pervious/permeable topsheet and a liquid impermeable and water vapor pervious/permeable backsheet. The absorbent core of the breast pads of the present invention preferably has a tensile strength under wet conditions of at least 550 g/25.4 mm. This advantageously permits that the pad maintains its integrity and hence does not disintegrate during use conditions. In the preferred embodiment herein the absorbent core comprises a fibrous matrix and a fibrous super absorbent gelling material (preferably at least 0.1% by weight of the absorbent core), and optionally a fibrous odor control material.

The breast pad according to the present invention may comprise a garment/bra facing surface of the breast pad (typically the outer surface of the backsheet) with a coefficient of static friction of at least 1 and/or a wearer facing surface of the breast pad (typically the outer surface of the topsheet) with a coefficient of static friction of at least 1. Typically only the garment/bra facing surface of the breast pad has a coefficient of friction of at least 1 for improved stay in place in the bra. The non-slip material might be applied on at least one outer surface of the breast pad in a continuous or preferably in a dispersed/discontinuous fashion (random or designed) for minimum impact on the breathability properties of the pad. Preferably the non-slip material used is water vapor pervious. In the execution herein wherein the non-slip material applied to the garment/bra facing surface of the breast pad is water vapor permeable, it can be advantageously applied in a continuous form without compromising on effective breathability properties of said surface.

## Brief description of the drawings

The invention is further described with reference to the accompanying drawing.

Figure 1 shows a cross sectional view of a preferred layer assembly composing a breast pad according to the invention.

Figure 2 shows a perspective view of a breast pad (circular shape) seen from the surface intended to face the garment/bra.

Figure 3 shows a preferred breast pad according to the present invention (having a geometry so that at least more than 50% of surface area of the pad is disposed beneath the breast nipple in use (triangular type shape)) seen from the surface intended to face the breast.

Figure 4 shows a cross sectional view of a breast pad of Figure 3 in section along the line I-I.

## Detailed description of the Invention

According to the present invention the breast pads for use herein are disposable breast pads.

The term 'disposable' is used herein to describe breast pads which are not intended to be laundered or otherwise restored or reused as a breast pad (i.e., they are intended to be discarded after a single use and, preferably to be recycled, composted or otherwise disposed of in an environmentally compatible manner).

As used herein, the terms "breast pad" refers to an absorbent article that is worn by women adjacent to the nipple region of a breast of a woman, and which is intended to absorb and contain fluid discharge from the woman's nipple and/or skin (perspiration).

By "fluid discharge" it is meant herein any fluid produced by the breast of nursing mothers during the latter stages of pregnancy and/or after child birth (commonly called lactational/milk discharge). Fluid discharge also includes perspiration. Indeed, the breast pads according to the present invention may also suitably be used by non nursing women suffering from perspiration problems during usual daily activities and/or from perspiration problems that might occur under stress conditions and/or during sport activities.

As used herein the term "wearer-facing" surface of the breast pad refers to the surface of the outer component of the breast pad oriented to directly face the wearer during use of the breast pad. As used herein the term "garment/bra-facing" surface of the breast pad refers to the opposite surface than the wearer facing surface, i.e. to the surface of the outer component of the breast pad oriented to directly face the bra/garment during use of the breast pad.

As used herein the term "granular" refers to materials with particles of uniform size or having a size distribution in which at least 5 % by weight of the particles have a largest diameter of at least 0.2 mm. The term "non-granular" excludes those materials which are granular.

Unless otherwise noted in the following all percentages refer to percent by weight.

The breast pads according to the invention have a wearer facing surface directed towards the wearer during use (typically the outer surface of the topsheet) and a garment facing surface directed towards a garment (usually a bra) during use (typically the outer surface of the backsheet). Typically the breast pads according to the present invention comprise a liquid permeable, water vapor pervious topsheet, a liquid impermeable, water vapor permeable backsheet and an absorbent core interposed between the topsheet and backsheet. Each of the elements of the breast pad can be selected from a wide variety of alternatives and may comprise several elements and/or layers contributing to the individual function of each elements provided that the conformability, breathability and optionally the fluid absorption capacity and/or stay in place properties and/or integrity properties under wet conditions of the pads herein are obtained.

The breast pads of the present invention offer enhanced comfort due to both their conformability properties and breathability properties. The comfort benefits are further enhanced in the preferred embodiment herein wherein the breast pads further deliver enhanced fluid absorption capacity, and/or enhanced stay in place properties without the need of conventional adhesive protected before use by release paper and/or enhanced integrity properties under wet conditions.

The breast pads according to the present invention are thin. Thinness of the pads contributes to comfort, pliability and flexibility properties of the breast pads. Advantageously thin breast pads are provided while not compromising on other properties like overall protection (absorption capacity and leakage prevention). Typically the overall thickness of the breast pad is of less than 5 mm, preferably less than 4.0, more preferably from 3.3 to 0.5 mm, even more preferably from 3.0 to 1.0 and most preferably from 2.9 to 1.5.

Advantageously the breathable breast pads herein in use conditions deliver less wetness on the skin, which is exposed to a more comfortable micro-climate (less humidity) resulting in higher skin comfort.

The breast pads according to the present invention have a water vapour permeability (as measured by the water vapour permeability test method described herein after) higher than 100 (g) / ($m^2$/24hrs), preferably of more than 200 (g) / ($m^2$/24hrs), more preferably higher than 300 (g) / ($m^2$/24 hrs), and most preferably higher than 400 (g) / ($m^2$/24 hrs).

The breast pads according to the present invention might also be air permeable. Typically they may have an air permeability (as measured by the air permeability test method described herein after) higher than 50 l/$m^2$/s, preferably higher than 100 l/$m^2$/s, and more preferably higher than 200 l/$m^2$/s.

The breast pads according to the present invention have a stiffness as determined per the modified ASTM D4032-94 test described herein after of less than 600 grams, preferably of less than 500 grams, more preferably of less than 400 and most preferably from 50 grams to 350 grams.

The stiffness of the breast pad can be measured according to a circular bend test which is based on a modified ASTM D4032-94 as described herein after. This circular bend test gives a force value expressed in grams related to resistance to bending, simultaneously averaging stiffness in all direction.

Advantageously the breast pads according to the present invention offer enhanced fit and comfort and a low degree of wearing awareness. Indeed the breast pads according to the present invention is highly flexible and conforms very well to the various anatomy of woman breast.

Typically the breast pads according to the present invention are provided with a effective fluid absorption capacity. In order to provide such effective absorbency with the thin pads according to the present invention the absorbency per surface area needs to be particularly high. The breast pads according to the present invention preferably have a fluid absorption capacity of at least 0.05 grams per $cm^2$. Typically the fluid absorption capacity of the structure expressed in grams per square cm of the breast pad is of at least 0.10 grams per $cm^2$, preferably from 0.15 to 1.50, more preferably from 0.20 to 0.80 and most preferably from 0.25 to 0.6 grams per $cm^2$.

Typically, the breast pad according to the present invention also has a total fluid absorption capacity of at least 4 grams per pad, preferably of at least 10 grams per pad, more preferably at least 15 grams per pad, even more preferably from 20 to 100 grams, and most preferably from 25 to 55.

The absorption capacity of the breast pads herein is measured according to modified ASTM D1117-80 'absorptive capacity test' described herein after. A suitable reference to take as the test fluid for performing this absorptive capacity test is saline water solution (0.9% NaCl in distilled water).

Preferably the absorbent core of the breast pads according to the present invention have high tensile strength

under wet conditions. In the preferred embodiments of the present invention the absorbent core typically has a wet-tensile strength in at least one direction when measured as described herein after by modified ASTM D5034-95 of at least 550 g/25.4mm, preferably from 1000 to 6000 g/25.4mm and more preferably from 1500 to 4000 g/25.4mm. It is understood herein that a tensile strength of at least 550 g/25.4mm under wet conditions for the absorbent core of the breast pad herein is preferably met in at least the machine direction or in at least the cross direction and more preferably in both directions.

In the following the elements of the breast pads are described.

Absorbent core

The absorbent core is a main component as far as stiffness, and hence pliability and flexibility of the breast pad is concerned. It is therefore essential that the absorbent core does not create a barrier to the comfort of the breast pad by having too high a bending resistance or resistance to conforming to its in-use situation, including the changes occurring during use. This can be achieved by appropriate material selection and/or treatment of the core as will be readily apparent to the skilled person. Typically the absorbent core also provides outstanding fluid absorption capacity and integrity in use, especially under wet conditions. Highly preferred absorbent cores for use in the breast pads herein to get at the same time optimum conformability, comfort, protection and integrity are those comprising fibrous matrix with non granular super absorbent gelling material, preferably fibrous super absorbent gelling material.

The absorbent core can be a single entity or comprise several layers. It can includes the following components: (a) optionally a primary fluid distribution layer; (b) optionally a secondary fluid distribution layer; (c) a fluid storage layer; (d) optionally a fibrous layer underlying the storage layer; and (e) other optional components.

a. Optional Primary Fluid Distribution Layer

One optional component of the absorbent core according to the present invention is the primary fluid distribution layer. This primary distribution layer typically underlies the topsheet (if present) and is in fluid communication therewith. The primary distribution layer is able to acquire fluid discharge for ultimate distribution to the storage layer. This transfer of fluid through the primary distribution layer occurs mainly in the thickness, but may also provide distribution along all transverse directions of the breast pad, thereby minimizing saturation in the central zone (i.e. nipple point) .

b. Optional Secondary Fluid Distribution Layer

Also optional according to the present invention is a secondary fluid distribution layer. This secondary distribution layer typically underlies the primary distribution layer and is in fluid communication therewith. The purpose of this secondary distribution layer is to readily acquire fluid discharge from the primary distribution layer and distribute it along all transverse directions of the breast pad before transfer to the underlying storage layer. This helps the fluid capacity of the underlying storage layer to be fully utilized.

c. Fluid Storage Layer

Positioned in fluid communication with, and typically underlying the primary or secondary distribution layers if present, is a fluid storage layer.

It typically comprises non-granular super-absorbent gelling materials usually referred to as "hydrogels," "superabsorbent" "hydrocolloid" materials. Absorbent gelling materials are those materials that, upon contact with aqueous fluids, like milk discharge and/or perspiration, imbibes such fluids and thus form hydrogels. These absorbent gelling materials are typically capable of absorbing large quantities of such fluid, and are further capable of retaining such absorbed fluids under moderate pressures. In the prior art these absorbent gelling materials are typically in a granular form of discrete, non fibrous particles. However, according to the present invention these super-absorbent gelling materials are preferably provided in non-granular form, more preferably in a fibrous form.

The fluid storage layer is made of any suitable fibrous matrix also referred to as carrier that typically further comprises absorbent gelling materials which can be dispersed homogeneously or non-homogeneously in said carrier. Suitable carriers include cellulose fibers, in the form of fluff, such as is conventionally utilized in absorbent cores. Modified cellulose fibers such as viscose/rayon, stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic and man-made fibers have from about 0.1 denier per filament to about 30 denier per filament, more preferably from about 0.5 denier

per filament to about 16 denier per filament. Preferred fibers used are so called long fibers with a cut length of at least 10 mm, more preferably at least 25 mm. The use of so called long fibers in the fibrous matrix of the storage layer are suitable herein to contribute to the integrity properties of the absorbent core, especially under wet conditions. Also preferably, the fiber surfaces are hydrophilic or are treated to be hydrophilic.

The storage layer can also include filler materials, such as Perlite, diatomaceous earth, Vermiculite, etc., that lower rewet problems, however, only in a non-granular form. Further the storage layer may comprise a binder including but not limited to latex binders which can be sprayed as an aqueous solution onto the surface of the storage layer prior to curing.

According to the present invention the breast pad have a stiffness as measured by modified ASTM D4032-94 test method described herein after of less than 600 grams. It is understood herein that the materials used and construction of pad is chosen so as to meet this criteria. This is particularly applicable to the absorbent core and hence storage layer used herein. Particularly suitable storage layer and hence fibrous matrix for use herein are those made of a process know as hydroentangling. General information about hydroentangling can be found for example in US 2,862,251 (Kalwaites), US 3,025,585 (Griswold), US 3,485,706 (Evans), US 5,204,158 (Phillips), US 5,320,900 (Oathout) and EP 418 493 (Fiberwet), the content of which are incorporated herein by reference. Typically in hydroentangling process a web of fibres, for example a carded web, travels beneath at least one array of orifices and preferably a plurality of from successive arrays, from which jets of water are emitted. Each array extends transversely with respect to the path of travel of the web, and provides a large number of closely spaced jets. These jets act like sharp needles, and entangle the fibres to form a substrate/fibrous matrix. This entanglement holds the fibres in a coherent substrate/fibrous matrix, without the need for adhesives or thermal bonding. Indeed in the preferred embodiments herein the storage layer is free of any binder/adhesives, the fibers being bonded by entangling them without binder/adhesives, thereby allowing very soft layer which deforms easily and appropriately conform to the 3D configuration of breasts. It should be mentioned that although hydroentangling is particularly suitable for use herein as the method of producing the fibrous matrix/storage layer, other methods known per se in the art can alternatively be used. For example water can be replaced by another liquid or by gas for example air or steam. Alternatively, the fluid 'needles' can be replaced by mechanical needles in a process known as needlepunching. In this barded needles, e.g. of steel, are punched through the web, hooking tufts of fibres across it and thereby bonding it in the needlepunched areas. The needles enter and leave the web while it is trapped between tow plates, the web being pulled through the apparatus by draw rolls. The web of fibers can be carded and crosslayed or airformed in order to achieve the desired weight and then consolidated by needlepunching. The level of entanglement is provided by the number of perforations per surface unit. Desired softness is achieved typically by changing the number of perforations per surface unit and/or the penetration of the needle through the web. It is by appropriately selecting the configuration of the needles, number of barbs, depth of barbs, and/or size of the working blade, which have a major role to play in the number of fibers carried within the web that the desired softness as well as other characteristics will be obtained.

A very soft storage layer, which is deformable/stretchable because the fibers are free to move and slip, are obtained by entangling given types of fibres with given length and denier while not loosing consistency, integrity because the fibers are still connected (entangled) even at high elongation. Highly preferred fibers or fibers blends used for such storage layer include rayon, lyocell or the like, wicking fibers like synthetic fibers such as polypropylene, polyester or others, and non granular preferably fibrous absorbent gelling materials.

If the absorbent gelling materials are dispersed non-homogeneously in a fibrous matrix, the storage layer can be locally homogeneous, i.e. have a distribution gradient in one or several directions within the dimensions of the storage layer. Non-homogeneous distribution thus includes e.g. laminates of the fibrous carriers enclosing the non-granular absorbent gelling materials.

Typically, the storage layer comprises from 0.1% to 95% non-granular absorbent gelling materials (preferably fibrous absorbent gelling materials), preferably from 5% to 80%, more preferably from 10% to 60% and most preferably from 15% to 55%. Further the storage layer can comprise from 5% to 99.9% of a fibrous matrix (also called herein carrier fibers), preferably from 95% to 20%, more preferably from 90% to 40% and most preferably from 85% to 45%. In the most preferred embodiment herein the fibrous matrix used is composed of a blend of 50% to 85% viscose fibers, preferably from 55% to 75% chosen in the range of 0.5 denier to 25 deniers, preferably between 1.2 denier and 2.5 deniers, with a cut length of 25mm to 100mm, preferably between 38 and 65mm. The fibrous absorbent gelling materials as for instance Camelot Fiberdri® are preferably used at a rate of 15% to 50%, preferably of 25% to 45%, selected in the range of 4 to 32 deniers, preferably at 9 to 10 denier, the length from 38mm to 75mm, preferably 40mm to 60mm.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 (Brandt et al), issued March 31, 1987, and reissued as RE 32,649 on April 19, 1988. Preferred monomers include acrylic acid, methacrylic acid, and 2-acrylamido-2-methyl propane sulfonic acid.

Acrylic acid itself is especially preferred for preparation of the super-absorbent material. The polymeric component formed from the unsaturated, acid-containing monomers can be grafted onto other types of polymer moieties such as starch or cellulose. Polyacrylate grafted starch materials of this type are especially preferred.

Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof. Especially preferred are the polyacrylates and polyacrylate grafted starch.

Whatever the nature of the basic polymer components of the hydrogel-forming polymeric absorbent gelling materials, such materials will in general be slightly crosslinked. Crosslinking serves to render the hydrogel-forming polymer gelling materials substantially water-insoluble, and cross-linking thus in part determines the gel volume and extractable polymer characteristics of the hydrogels formed from these polymeric gelling materials. Suitable crosslinking agents are well known in the art and include, for example, those described in greater detail in U.S. Patent 4,076,663 (Masuda et al), issued February 28, 1978. Preferred crosslinking agents are the di- or polyesters of unsaturated mono- or polycarboxylic acids with polyols, the bisacrylamides and the di- or triallyl amines. Other preferred crosslinking agents are N,N'-methylenebisacrylamide, trimethylol propane triacrylate and triallyl amine. The crosslinking agent can generally constitute from about 0.001 mole percent to 5 mole percent of the resulting hydrogel-forming polymer material. More preferably, the crosslinking agent will constitute from about 0.01 mole percent to 3 mole percent of the hydrogel-forming polymeric gelling material.

The slightly crosslinked, hydrogel-forming polymeric gelling materials are generally employed in their partially neutralized form. For purposes of the present invention, such materials are considered partially neutralized when at least 25 mole per-cent, and preferably at least 50 mole percent of monomers used to form the polymer are acid group-containing monomers that have been neutralized with a salt-forming cation. Suitable salt-forming cations include alkali metal, ammonium, substituted ammonium and amines. This percentage of the total monomers utilized which are neutralized acid group-containing monomers is referred to herein as the "degree of neutralization."

While these absorbent gelling materials have typically been disclosed in the prior art in granular form, it is highly preferred in the present invention that the absorbent gelling material is in a non-granular form for example as macrostructures such as fibers, sheets or strips. These macrostructures can be prepared by forming the particulate absorbent gelling material into an aggregate, treating the aggregated material with a suitable crosslinking agent, compacting the treated aggregate to densify it and form a coherent mass, and then curing the compacted aggregate to cause the crosslinking agent to react with the particulate absorbent gelling material to form a composite, porous absorbent macrostructure. Such porous, absorbent macrostructures are disclosed, for example, in U.S. Patent 5,102,597 (Roe et al), issued April 7, 1992.

More generally any super-absorbent fiber known in the art may be used as a super-absorbent fiber in the absorbent core. Superabsorbent fibers can be made by forming a water soluble super-absorbent polymer into water soluble filaments, contacting the filaments with a primary air stream having a velocity effective to attenuate and to partially dry the filaments, and contacting the attenuated filaments with a secondary air stream having a velocity effective to fragment the filaments into fibers. Particularly suitable super-absorbent polymers are polymers comprising a blend of

- a copolymer of at least one alpha, beta-unsaturated carboxylic monomer and at least one monomer copolymerizable therewith, and

- a cross-linking agent having cross-linking functionality comprising hydroxyl or heterocyclic carbonated groups. Preferred are maleic anhydride/isobutylene copolymers cross-linked with propylene carbonate or a mixture of pentaerythriol and butanediol.

An example of a super-absorbent fiber embodiment of the present invention is Fibersorb, a commercially available superabsorbent fiber from Arco Chemical Company of Newton Square, Pennsylvania. These fibers are disclosed more fully in U.S. Patent No. 4,855,179, issued August 8, 1989, to Bourland et al. Alternatively such super-absorbent fibers can be available under the trade name FIBERDRI®, commercially available from CAMELOT TECHNOLOGY LTD.

In the preferred embodiment of the present invention the breast pad according to the present invention comprises a topsheet, a backsheet and an absorbent core between said topsheet and backsheet, said absorbent core comprising a fibrous matrix and non granular super absorbent gelling material, preferably fibrous super absorbent gelling material (as the fluid storage layer). The presence of such an absorbent core has the advantage of providing breast pads being thin (less than 5 mm) with enhanced conformability (defined herein by stiffness of less than 600 grams), effective absorption capacity, and enhanced integrity (defined herein by core tensile strength under wet conditions), i.e., breast pads combining at the same time the consumers noticeable advantages of discretion in use, comfort and protection.

Advantageously the use of such non-granular (fibrous) super absorbent gelling materials instead of granular

super absorbent gelling materials is particularly safe for the babies. Indeed the occurrence of accidental particulate or granular materials lost that might otherwise typically arise through apertured topsheet of the breast pads of the art is avoided. Also the use of such non-granular (fibrous) super absorbent gelling materials results in manufacturing ease with reduced spillage occurrence and dust occurrence.

d. Optional Fibrous Layer

An optional component for inclusion in the absorbent cores according to the present invention is a fibrous layer adjacent to, and typically underlying the storage layer. This underlying fibrous layer would typically provide the same function as the secondary fluid distribution layer.

e. Other Optional Components

The absorbent cores according to the present invention can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. Given the structural integrity that usually occurs as a result of thermal bonding, reinforcing scrims are usually not required for the breast pads according to the present invention.

Other components which can be included in the absorbent core according to the invention and preferably are provided close to or as part of the primary or secondary fluid distribution layer or can also be dispersed homogeneously or non-homogeneously within the storage layer or between the storage layer and underlying layer like a layer of the backsheet are odor control agents. These components if present are preferably incorporated in non-granular form too.

Any odour control agent or combinations thereof, known in the art for this purpose may be used herein. These agents can typically be classified according to the type of odour the agent is intended to combat. Odors may be chemically classified as being acidic, basic or neutral. Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas, and starches.

Typically, the absorbent core may comprise the odor control agent or a mixture thereof at a level of from 0 $gm^{-2}$ to 600 $gm^{-2}$, preferably from 0.1 to 500 $gm^{-2}$, more preferably from 1 $gm^{-2}$ to 350 $gm^{-2}$ and most preferably from 5 $gm^{-2}$ to 200 $gm^{-2}$

Physical characteristics of absorbent cores

Absorbent cores are usually non extensible and non-elastic, however, they can be rendered extensible and depending on the selected materials can also be made to have elastic characteristics. The term "extensible" as used hereinafter refers to a structure which under external forces such as those occurring during use of a breast pad extends in the direction of the forces or in the direction of a component of the forces in cases where only mono directional extensibility is provided.

The term "elastic" as used hereinafter refers to extensible structures which return at least partially to their initial state after the forces causing the extension seize to be exerted. Absorbent cores can be corrugated or pleated in one or several directions to provide a certain extensibility while selection of elastic fibers for the structure can provide elasticity.

The absorbent cores should preferably be thin. A thickness of less than 3 mm, preferably less than 2 mm is desirable such that the resulting breast pads have a thickness of less than 5.

Topsheet

The topsheet if present is compliant, soft feeling, and non-irritating to the wearer's skin. The topsheet also can have elastic characteristics allowing it to stretch in one or two directions or even preferably in all directions. Further, the topsheet is fluid pervious permitting fluids (e.g., milk discharge/perspiration) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and aperture formed film topsheets. At least in the region where fluid is expected to be discharged onto the breast pad the apertured formed films are preferred because they are pervious to milk discharge/fluid and yet non-absorbent and have a reduced tendency to allow fluids to pass back through and rewet the wearer's skin. Thus, the surface of the formed film that is in contact with the breast's skin remains dry, thereby reducing skin soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Particularly preferred microapetured formed film topsheets are disclosed in U.S. patent 4,609,518 (Curro et al), issue September 2, 1986 and U.S. patent 4,629,643 (Curro et al), issued December 16, 1986. One preferred topsheet for the present invention is the formed film described in one or more of the above patents and marketed on sanitary napkins by The Procter & Gamble Company of Cincinnati, Ohio as "DRI-WEAVE."

In one embodiment of the invention the topsheet can be a so-called hybrid topsheet in which the wearer contacting surface is provided in its center (i.e., region intended to directly face the nipple of the breast) by an apertured formed film while a region surrounding the center is provided with a non-woven such as e.g. the high loft non-woven mentioned above or other non-woven which does provide particularly skin friendliness. Such topsheets have been disclosed for pantiliners in EP-A-523 683, EP-A-523 719, EP-A-612 233, or EP-A-766 953 and are applicable in a circular design to breast pads needs.

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in PCT Publication No. WO93/09741, "Absorbent Article Having A Nonwoven and Apertured Film Coversheet" filed on November 19, 1991 by Aziz, et al. Alternatively, the body surface of the topsheet can be made hydrophilic by treating it with a surfactant such as is described in the above referenced U.S. 4,950,254.

Backsheet

The backsheet prevents the fluid absorbed and contained in the absorbent core from wetting articles that contact the breast pad such as bra/undergarments. Preferably the backsheet is impervious to liquids (e.g., milk discharge and/or perspiration). It can be manufactured from a modified thin plastic film, although other flexible liquid impervious materials can also be used. As used herein, the term "flexible" refers to materials that are compliant and will readily conform to the general shape and contours of the breasts. The backsheet preferably also can have elastic characteristics allowing it to stretch in one or two directions or even preferably in all directions.

The breast pads according to the present invention have a breathable backsheet which is water vapor permeable, i.e., typically the backsheet has water vapor transfer rate of at least 100 $g/m^2 \cdot 24h$, more preferably at least 200 $g/m^2 \cdot 24h$, and most preferably at least 300 $g/m^2 \cdot 24h$.

a. Water vapor permeable composite structure comprising a hydrophilic thermoplastic film

In a preferred embodiment herein the breast pads have a backsheet made of a water vapor permeable liquid impervious composite structure comprising a hydrophilic thermoplastic film and a support layer.

By 'hydrophilic thermoplastic film' it is meant herein a continuous film that do not allow the flow of water vapor through open pores or apertures in the material, but do transfer substantial amounts of water vapor through the film by absorbing water on one side of the film where the water vapor concentration is higher, and desorbing or evaporating it on the opposite side of the film where the water vapor concentration is lower.

According to the present invention the composite structures comprise a hydrophilic thermoplastic film made from a water vapor permeable thermoplastic composition.

Suitable thermoplastic compositions for use herein are thermoplastic compositions known to those skilled in the art for making hydrophilic continuous water vapor permeable, liquid impermeable layers or films ("monolithic films") having the characteristics of water vapor permeability and liquid imperviousness.

Typically the thermoplastic film of the composite structures for use according to the present invention is made

from a water vapor permeable thermoplastic composition which comprises as an essential component from 5% to 100% by weight of said film of a polymer or mixture of polymers. More preferably the thermoplastic composition comprises from 10% to 80% and most preferably from 25% to 70% by weight of said polymer.

The thermoplastic compositions for use herein typically comprise thermoplastic polymers such as polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof.

Particularly preferred thermoplastic polymers are thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes (e.g. Estane™), or mixtures thereof.

Such thermoplastic polymers or mixture of polymers can be typically highly viscous in the melted state at the process conditions that are typical of the known processes of film or layer formation, e.g. an extrusion process involving a high power screw extruder. For example they may have a viscosity higher than 5000 poise at a temperature of 20°C above the DSC (Differential Scanning Calorimetry) melting point, which is the temperature identified as that corresponding to the DSC peak, or corresponding to the highest DSC peak in case of a mixture of polymers showing more than one peak, and at a frequency of 1 rad/sec.

The viscosity of the preferred thermoplastic polymers or mixture of polymers is adjusted by including in the thermoplastic composition a suitable plasticiser, or blend of plasticisers, that is compatible with the thermoplastic polymer(s) and that lowers the viscosity of the thermoplastic polymer or mixture of polymers in the melted state.

The thermoplastic compositions for use herein comprising the suitable plasticiser or blend of plasticisers have the following complex viscosities ($\eta*$ ):

50 poise < $\eta*$ < 4000 poise, preferably 100 poise < $\eta*$ < 2000 poise, more preferably 100 poise < $\eta*$ < 1000 poise, at a frequency of 1 rad/s at a temperature of 210°C or less and $\eta*$ < 2000 pose, preferably $\eta*$ < 1000 poise, more preferably $\eta*$ < 500 poise, at a frequency of 1000 rad/s at a process temperature (T) of 210°C or less, wherein $\eta*$ represents the complex viscosity of the thermoplastic polymeric composition. Preferably the temperature T is 200°C or less and more preferably 180°C or less and most preferably from 200°C to 50°C.

The thermoplastic compositions having the complex viscosity described allow for a film or layer to be coated onto a support layer/substrate using typical coating conditions and apparatuses known in the art for the coating of low viscosities hot melt compositions in a layer having a required thickness onto a support layer/substrate, while also keeping the advantageous characteristics of the preferred thermoplastic polymers in providing hydrophilic continuous water vapor permeable, liquid impermeable layers or films.

Thermoplastic compositions having such viscosities can also provide very thin films or layers. Typically the thermoplastic film used in the composite structures of the present invention have a thickness of less than 200 microns, preferably less than 130 microns, more preferably less than 80 microns, even more preferably from 70 to 0.5 microns and most preferably from 55 to 2 microns.

Advantageously the films used herein allow for films to be produced at greatly reduced thickness, whilst maintaining the mechanical properties of the film such that no failures concerning the uniformity of the film such as breakages or the presence of apertures arise.

Suitable plasticisers or blend of plasticisers for adjusting such viscosity can be selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids ($C_8$-$C_{22}$) and their derivatives, and mixtures thereof. This allows to utilise with these preferred compositions typical process conditions known in the art for the direct coating of low viscosity hot melts onto a substrate in order to form a water vapor permeable, liquid impervious film or layer.

It has also been found that by further selecting the plasticiser or blend of plasticisers to be comprised in the thermoplastic composition used herein from the group of hydrophilic plasticisers consisting of acids, esters, amides, alcohols, polyalcohols, or mixtures thereof, the advantage of an enhanced water vapor permeability of the resulting layer or film formed from the thermoplastic composition is also achieved, when compared to a corresponding film or layer formed from a thermoplastic composition comprising the same thermoplastic polymer, but without such a plasticiser.

The preferred hydrophilic plasticiser or blend of hydrophilic plasticisers can of course also adjust the viscosity of the thermoplastic composition for use herein to the preferred values in order to make it processable by coating said thermoplastic composition onto a substrate in a layer or film having a desired thickness.

Suitable preferred hydrophilic plasticisers according to this preferred embodiment herein are citric acid esters,

tartaric acid esters, glycerol and its esters, sorbitol, glycolates, and mixtures thereof.

Thus in addition to the thermoplastic polymeric component, the thermoplastic compositions also preferably further comprise a plasticiser or blend of plasticisers, preferably from 0% to 95%, more preferably from 20% to 90%, most preferably from 25% to 75% by weight of said thermoplastic composition.

The thermoplastic compositions from which the polymeric films of the composite structures of the present invention are made may in addition comprise additional optional components to further improve the processibility of the film and mechanical characteristics as well as other characteristics as its tackiness, its resistance to aging by light and oxygen, its visual appearance etc.

Such optional components include tackifying resins or blends of tackifying resins having a softening point of 125°C or less. Preferred resins, which may be present by up to 50% by weight, may be selected from rosins and rosin esters, hydrocarbon resins, aliphatic resins, terpene and terpene-phenolic resins, aromatic resins, synthetic $C_5$ resins, mixtures of synthetic $C_5$-$C_9$ resins, and mixtures thereof.

In a particular embodiment herein wherein it is desirable to provide increased friction properties of the thermoplastic film this can be achieved by suitably selecting the tackifier resin, or blend of tackifier resins, to be added to the thermoplastic composition. Indeed, it is possible to adjust the residual tackiness of said thermoplastic composition at room temperature. This in turn allows an adjustment of the friction that is established, in the use conditions, between the layer made of said thermoplastic composition comprised in the composite structure and any other layers (typically the support layer and typically another layer for example the core of the breast pad, the thermoplastic film having two opposing surface one facing the directly the core of the breast pad and one facing the support layer). In another embodiment of the present invention the thermoplastic film may face a wearer's garment (when used as the outer surface facing the wearer's garment). An increased friction provides in fact a better stability of the liquid impermeable, water vapor permeable thermoplastic film with respect to surface layer to which it is applied, avoiding or at least reducing the relative movements.

By suitably selecting the tackifier resin or blend of tackifier resins for use herein it is also possible to adjust the residual tackiness at room temperature of the thermoplastic composition, and therefore of the layer formed from said thermoplastic composition, to the extent that it has the typical characteristics of a pressure sensitive adhesive. In other words, the friction between the layer of thermoplastic composition comprised in the composite structure of the present invention can be increased such that it can actually stick to the surface to which it is applied to, owing to the thermoplastic composition which can be formulated in order to behave like a pressure sensitive adhesive. Residual tackiness of the thermoplastic composition at room temperature can be suitably tailored since a smooth transition exists between increasing friction and actual adhesiveness.

Accordingly particularly preferred tackifier resins, or blend of tackifier resins to be selected herein have a softening point of 125°C or less, and are selected from the group consisting of rosin and rosin esters, terpene-phenolic resins, aromatic resins, and mixtures thereof.

Most preferably, the thermoplastic compositions for use herein comprise a blend of tackifier resins, preferably selected as described above, wherein moreover from 0% to 20%, preferably from 2% to 15%, by weight of said blend of tackifier resins comprises a resin or blend of resins having a softening point of less than 25°C, and from 80% to 100%, preferably from 85% to 98%, by weight of said blend of tackifier resins comprises a resin or blend of resins having a softening point of at least 70°C.

Particularly suitable tackifier resins for use herein are those described in our European patent application No. 00116284.1, filed on 10 August 2000 with title: "Thermoplastic hydrophilic polymeric compositions with improved adhesive properties for moisture vapour permeable structures" (P&G Case CM2406F).

The presence in the blend of tackifier resins of a certain amount of one or more tackifier resins which are liquid at room temperature is preferred since said resin or resins, in addition to adjusting the residual tackiness of the layer formed from the resulting thermoplastic composition, also contribute to lower the viscosity of the composition itself at the process conditions, in combination with the selected suitable plasticiser or blend of plasticisers.

Said preferred blend of tackifier resins therefore provides a thermoplastic composition for forming the thermoplastic film in the composite structures of the present invention, which is easily processable. Moreover the preferred blend of tackifier resins allows to adjust the residual tackiness of the resulting thermoplastic film made from the thermoplastic composition, and therefore the friction properties towards the surface it is applied to as desired.

The thermoplastic compositions for making the thermoplastic film of the composite structures described herein typically comprises from 0% to 60%, preferably from 2% to 60%, more preferably from 5% to 50%, and most preferably from 10% to 40%, by weight of said thermoplastic composition, of a tackifier resin or blend of tackifier resins.

Other optional components include anti-oxidants, anti-ultraviolets, pigments and mixtures thereof, which may be present within the thermoplastic composition at a level of up to 10% by weight of the composition.

A thermoplastic composition used herein can be manufactured with a process that will typically comprise the steps of providing the thermoplastic polymer or mixture of polymers, the optional plasticiser or blend of plasticisers, and the optional tackifier resin or blend of tackifier resins, heating the components and compounding them, e.g. with

a known suitable mixer to form the thermoplastic composition in the molten state having the desired complex viscosity η*.

A water vapor permeable, liquid impervious composite structure can be formed from the thermoplastic composition described herein by coating said thermoplastic composition onto a support layer/substrate. The films (also called layers) formed from the thermoplastic compositions of the present invention preferably have a water vapor transport rate of at least 100 $g/m^2 \cdot 24h$, preferably at least 300 $g/m^2 \cdot 24h$, most preferably at least 500 $g/m^2 \cdot 24h$ with a thickness of the layer or film of at least 0.5 μm.

A process for making a layer or film from a thermoplastic composition as described herein typically comprises the steps of providing said composition, heating it to make it flowable, and coating said composition in the molten state onto a substrate in a layer having the desired thickness. While said substrate can be simply a formation substrate, onto which the thermoplastic composition is coated in order to form a film or layer of the desired thickness which is subsequently separated from said substrate and used as such, in a preferred embodiment of the present invention a water vapor permeable, water impervious composite is formed which comprises the thermoplastic composition and a support layer/substrate onto which said thermoplastic composition is coated, wherein the support layer/substrate is also water vapor permeable.

Such embodiment provides a water vapor permeable, liquid impervious composite structure wherein the contribution of the film formed from the thermoplastic composition described herein to the performance of the composite material resides only in the provision of a liquid barrier and hence could be advantageously provided as thinly as possible. The remaining performance physical criterion being preferably provided by the provided structure, that therefore preferably acts also as a support layer.

The substrate, or support layer may be any useful layer which is also water vapor permeable, preferably having a water vapor permeability of at least 100 $g/m^2 \cdot 24h$, more preferably at least 300 $g/m^2 \cdot 24h$, and most preferably at least 500 $g/m^2 \cdot 24h$.

Suitable support layer for use herein include two dimensional, planar micro and macro-porous films; macroscopically expanded films; formed apertured films; nonwoven and woven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two dimensional porous planar layers may be made of any material known in the art, but are preferably manufactured from commonly available polymeric materials. Suitable materials are XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use as the support layer may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Preferred support layers for use herein include woven and nonwoven layers.

The composites described herein are particularly advantageous as they allow the possibility of providing a composite wherein the thermoplastic composition may be coated onto the support layer/substrate as a layer with the desired thickness. Typical coating conditions and apparatuses known in the art for the direct coating of low viscosities hot melts can be readily utilized in order to provide the thermoplastic composition at the desired thickness.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate.

The above described backsheets are preferred herein as they are extremely thin and are not impairing on

the conformability of the absorbent core like those comprising fibrous matrix and fibrous super absorbent gelling materials. It has now surprisingly been found that it is by combining these materials that optimum comformability (i.e., reduced stiffness) and comfort is achieved while not compromising on the fluid absorption capacity and even improving it.

Combining the absorbent core with fibrous matrix and fibrous super absorbent gelling materials with the above described composite structure (used as preferred backsheet of the breast pads herein) further contributes to the integrity of such composite structure with reduced risk of rupture of such composite structure, that might otherwise occur with granular super absorbent gelling materials.

b. <u>Other backsheet</u>

In another embodiment herein the breast pads are also air permeable. Typically the breast pads herein might have an air permeability (as measured by the air permeability test method described herein after) higher than 50 $l/m^2/s$, preferably higher than 100 $l/m^2/s$, and more preferably higher than 200 $l/m^2/s$.

In addition to water vapor permeability, the air permeability might be desirable in order to further improve the comfort benefit of breast pads herein.

Suitable backsheets for use herein to deliver air permeability are breathable backsheets comprising apertures.

The backsheet can comprise a woven or nonwoven material, polymeric films such as thermoplastic films of polyethylene or polypropylene, or composite materials such as a film-coated nonwoven material or fiber coated film. Preferably, the backsheet comprises a modified polyethylene film having a thickness of from about 0.012mm to about 0.1mm and more preferably from 0.002mm to 0.07mm.

The backsheet is preferably embossed and/or matte finished to provide a more clothlike appearance (unless the outer surface is provided by a woven or non-woven material as such). The backsheet can also comprise more than one breathable layer so as to replace a single breathable backsheet layer by at least 2 or 3 layers of a different or the same material. In particular two breathable layers forming together the breathable backsheet are preferred.

In an embodiment of the present invention the breathable backsheet comprises a hydrophobic, gas-permeable fibrous fabric layer composed of polymeric fibers, which is substantially liquid impervious. Such fibrous layers have inherent breathability apertures but in addition may be provided with apertures such as the film described below.

The fibrous fabric layer useful in the present invention preferably has a basis weight of 10 to 100 $g/m^2$ preferably 15 to 50 $g/m^2$. The fibres can be made of any hydrophobic polymeric material usual in the art of making fibrous fabric layers. Depending on the circumstances of the ultimate use and manufacturing of the breathable breast pad, fibres of polyethylene, polypropylene, polyester, polyacetat or combinations thereof (intra- and inter-fibres combinations) have been found useful.

The fibres are spunbonded, carded, melt blown or combinations thereof. The fabric layer might be made of a carded nonwoven. Alternatively, the fabric layer might comprises a matrix of spunbonded fibres covered on one or both sides with meltblown fibres but can also be provided by any other typical technology used in the art. If two or more fibrous fabric layers of different fiber thickness are used it is most preferred to have the one with the higher fiber thickness on the outer surface of the breast pad.

In an alternative embodiment of the present invention the breathable backsheet comprises a hydrophobic, gas-permeable apertured polymeric film. This film preferably has a first liquid transport direction and a second liquid transport direction opposite the first liquid transport direction and is oriented such that the first direction is from the backsheet towards the absorbent core. This directional apertured film allows a liquid transport in the first liquid transport direction which is larger than the liquid transport in the second liquid transport direction under an identical pressure drop across said apertured film. Most preferably the film comprises funnel shaped apertures wherein the direction from the larger funnel opening towards the smaller funnel opening is parallel to the direction from the garment facing side of the backsheet to the wearer facing side.

The apertured film also useful for embodiments of the present invention can be any of those well known in the art. This includes in particular, but is not limited to those films disclosed in U.S. 3,929,135, U.S. 4,151,240, U.S. 4,319,868, U.S. 4,324,426, U.S. 4,342,314, U.S. 4,591,523 and U.S. 4,609,518, U.S. 4,629,643, U.S. 5,158,819, U. S. 4,772,444.

The apertured film comprised in the inner layer of the breathable backsheet preferably has funnel shaped apertures similar to those described e.g. in US 3,929,135. The apertures maybe circular or non-circular but have a cross sectional dimension at one end of the funnel which is wider than the opening at the other end of the funnel. The direction from the larger funnel opening towards the smaller opening is of course generally parallel to the first liquid transport direction. The open area of the apertured film is typically more than 5%, preferably in the range of 10% to 35% of the total film surface. The apertured films can be made of any material typical in the art but preferably is made of a polymer similar to those used for the fibrous fabric layer.

The minimum hydraulic diameter of the apertures in the film should be as small as possible while still providing

sufficient gas permeability without hydraulic blockage of the apertures. A hydraulic diameter of as little as 0.1mm, preferably 0.2 to 0.7mm has been found possible in the context of the present invention. Hydraulic diameter for non circular apertures is the diameter that a circular aperture with the same cross section would have. Diameter is always determined in the plane of smallest cross section of an aperture.

In a particularly preferred embodiment herein a dual or multiple layer breathable backsheet composite is used in the breast pad. According to the present invention suitable breathable backsheets for use herein comprise at least a first and a second layer. The first layer is positioned between the garment facing surface of the absorbent core and the wearer facing surface of the second layer. It is oriented such that it retards or prevents liquid from passing from the absorbent core towards the outside while allowing free air flow and water vapor through it. The second layer provides water vapor and air permeability so as to support breathability of the pad.

Such a first layer is preferably in direct contact with the absorbent core. It provides air and water vapor permeability by being apertured. Preferably this layer is made in accordance with the aforementioned US-A-5,591,510 or PCT WO-97/03818, WO-97/03795. In particular, this layer comprises a polymeric film having capillaries. The capillaries extend away from the wearer facing surface of film at an angle which is less than 90 degrees. Preferably the capillaries are evenly distributed across the entire surface of the layer, and are all identical. However, layers having only certain regions of the surface provided with apertures, for example only an area outside the region aligned with the central loading zone of the absorbent core (e.g. nipple circular zone), maybe provided with such capillaries.

Methods for making such three-dimensional polymeric films with capillary apertures are identical or similar to those found in the apertured film topsheet references, the apertured formed film references and the micro-/macroscopically expended film references cited above. Typically a polymeric film such as a polyethylene (LDPE, LLDPE, MDPE, HDPE or laminates thereof) or preferably a monolithic polymeric film is heated close to its melting point and exposed through a forming screen to a suction force which pulls those areas exposed to the force into the forming apertures which are shaped such that the film is formed into that shape and, when the suction force is high enough, the film breaks at its end thereby forming an aperture through the film.

Especially using a monolithic polymer film as the material for the first layer provides water vapor permeability even under stress conditions. While the apertures provide air permeability during "leakage safe" situations but close the capillaries under stress conditions the monolithic material maintains water vapor permeability in such a case. Preferred breathable monolithic film materials for use herein are those having a high vapor exchange. Suitable monolithic films include Hytrel (TM), available from DuPont Corporation, USA, and other such materials as described in Index 93 Congress, Session 7A "Adding value to Nonwovens", J-C. Cardinal and Y. Trouilhet, DuPont de Nemours international S.A, Switzerland.

Various forms, shapes, sizes and configurations of the capillaries are disclosed in EP-A-934 735 and EP-A-934 736 both of which are herein incorporated for reference. In particular the apertures form capillaries which have side walls. The capillaries extend away from the wearer facing surface of the film for a length which typically should be at least in the order of magnitude of the largest diameter of the aperture while this distance can reach up to several times the largest aperture diameter. The capillaries have a first opening in the plane of the garment facing surface of the film and a second opening which is the opening formed when the suction force (such as a vacuum) in the above mentioned process creates the aperture. Naturally the edge of the second opening may be rugged or uneven, comprising loose elements extending from the edge of the opening. However, it is preferred that the opening be as smooth as possible so as not to create a liquid transport entanglement between the extending elements at the end of the second opening of the capillary with the absorbent core in the breast pad (in contrast this may be desirable for apertured film topsheets where such loose elements provide the function of sucker feet). The capillaries in the first layer of the breathable backsheet allow air and water vapor permeability which is not hindered by them being slanted at an angle or by the shape. At the same time the slanting and shaping will allow the capillaries to close under pressure excerpted from the wearer facing side on them such that liquid transport through the capillaries towards the outside of the pad becomes nearly impossible. Hence these three-dimensional formed film layers are highly preferable in the context of breathable breast pads and in particular so with the additional second outer layer which is provided as hereinafter explained.

In such a preferred embodiment the second outer layer of the breathable backsheet is a fibrous nonwoven web having a basis weight of less than 50g/m2, preferably of less than 40g/m2. Alternatively, the second outer layer is a fibrous nonwoven web formed by a layered composite of a meltblown nonwoven layer made from synthetic fibers having a basis weight of less than 13g/m2 and of a spunbonded nonwoven layer also made from synthetic fibers.

In the most preferred embodiment herein the backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer of a porous web which is a fibrous nonwoven composite web having a basis weight of less than50 g/m2 and preferably less than 40.

Using as the breathable backsheet in the breast pad of the present invention, a backsheet comprising at least

one breathable layer of a resilient, three dimensional web which consists of a liquid impervious polymeric film having apertures forming capillaries which are not perpendicular to the plane of the film but are disposed at an angle of less than 90° relative to the plane of the film, and at least another breathable layer of a porous web which consists of a fibrous nonwoven web having a basis weight of less than 50g/m2 (particularly of about 35g/m2), further contributes to the outstanding benefit of the present invention. Indeed these backsheet functions very well in term of comfort, soiling of the user garment, dryness, etc. while providing additional comfort due to the reduced basis weight of the non-woven layer. This reduction of basis weight also provides an improved material consumption structure of the whole pad.

Micro-porous backsheets may suffer from the use of particles, especially large particle size materials. The reason is that this micro-porous materials are often very soft and pliable due to their manufacturing process in which conventionally a polyethylene is mixed with a calcium carbonate and the film thereafter is drawn such that the calcium carbonate, which is incompatible with the polyethylene, introduces locations where cracks are formed through the film such that these cracks form micro pores which allow water vapor to permeate through the film.

These particularly pliable and soft materials will, however, also transmit localized forced peaks such as can be created by particles. Therefore, the use of particles in the breast pads using a micro-porous, water vapor permeable, breathable backsheet film could create discomfort due to the closeness of wearing such breast pads. Also, any particle pressing against the micro-porous backsheet may create a local aperture which is not desirable for backsheets. This is thus another reason to preferably use non granular super absorbent gelling materials.

Micro-porous films are well-known in the art and many disclosures of micro-porous films have been made. A list of processes for preparing micro-porous films and the use of micro-porous films in absorbent articles in general can be found for example in GB-A-1,452,977, US 4,347,844, US 4,091,164, US 4,153,751, US 4,698,372, US 4,815,714, US 4,923,650, US 5,008,296, US 5,011,698, US 5,409,761. Micro-porous films can be obtained for example from the Exxon Company, USA, or the Mitsui Company, Japan.

<u>Optional ingredients</u>

Other components which can be included in the breast pad herein includes chitin and/or chitosan materials. By 'chitosan material' it is meant herein chitosans, modified chitosans, crosslinked chitosans and chitosan salts. Chitosan materials are available in powder form and fibrous form. Preferably for use herein chitosan material is used in fibrous form when added within the absorbent core. Chitosan materials might also be used herein in any location of the breast pad for example within the topsheet and/or between the topsheet and the absorbent core and/or between the absorbent core and backsheet.

Chitosan materials have been found to be particularly suitable for breast pads as an effective material for controlling milk/lactational fluid discharges (including absorbing and retaining such fluid discharges typically by gelifying it within the pad). The use of chitosan materials in breast pads provides effective fluid absorption towards lactational fluids/milk discharges, i.e. fluids containing a high proportion of electrolytes and proteins that typically would have the tendency to interfere with conventional gelling absorbent materials like polyacrylates. Also the antimicrobial activity of chitosan material prevents the formation of skin irritation or even breast infection while being safe to babies. Finally the odour control properties of chitosan materials control odour associated with such lactational fluids.

Advantageously the presence of a chitosan material (preferably a chitosan salt like chitosonium pyrrolidone carboxylate), in a breathable breast pad according to the present invention provides not only initial comfort but maintain a higher level of comfort during use while providing at the same time high level of protection. Chitosan materials have the ability to instantaneously reduce fluid diffusion once they are contacted with fluids, thereby concentrating the storage of acquired fluid in their close proximity. This reduction of internal liquid transport results in reduced surface area of the absorbed fluid (e.g., reduced surface area of the stains of milk discharge in the pads). In other words the presence of a chitosan material in a breathable pad will result in larger area of dry pad which is not soiled by fluids. Advantageously the concentration of the fluid at limited locations on the pad will maintain the overall breathability of the pad at higher level in comparison to the same pad subjected to the same amount of fluid but wherein the fluid is left to diffuse over bigger surface area.

Without to be bound by any theory, this benefit is obtained due to the properties of chitosan material to instantaneously gelify lactational fluids coming into contact with it. The gelification rate of chitosan material is only a few seconds towards such fluids, i.e., organic fluids. The positively charged cationic groups of the chitosan materials will interact with negatively charged anionic functionalities present in fluids, like the carboxylic groups of proteins or hydroxylic groups of short chain acids (e.g., butyric acid). This will result in the formation of tridimensional net between cationic function of the chitosan materials and such molecules with anionic groups. This rapid physical change of the milk fluid will instantaneously immobilize it in the pad avoiding fluid transfer.

Chitosan is a partially or fully deacetylated form of chitin, a naturally occurring polysaccharide. Indeed, chitosan is an aminopolysaccharide usually prepared by deacetylation of chitin (poly-beta(1,4)-N-acetyl-D-glucosamine).

Chitin occurs widely in nature, for example, in the cell walls of fungi and the hard shell of insect and crustaceans. The waste from shrimp-, lobster, and crab seafood industries typically contains about 10 to about 15 percent chitin and is a readily available source of supply. In the natural state, chitin generally occurs only in small flakes or short fibrous material, such as from the carapace or tendons of crustaceans. There is generally no source, as with cotton in the cellulosics, that forms useful shaped articles without solution and re-precipitation or re-naturing

More specifically, chitin is a mucopolysaccharide, poly-N-acetyl-D-glucosamine with the following formula:

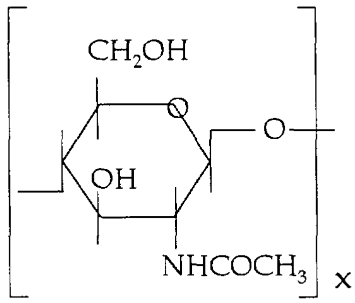

wherein x represents the degree of polymerization. Although x cannot be determined precisely, x is believed to be commonly in the range of from about 30 to about 50,000.

Chitosan is not a single, definite chemical entity but varies in composition depending on the conditions of manufacture. It may be equally defined as chitin sufficiently deacetylated to form soluble amine salts. Chitosan is the beta-(1-4) polysaccharide of D-glucosamine, and is structurally similar to cellulose, except that the C-2 hydroxyl group in cellulose is substituted with a primary amine group in chitosan. The large number of free amine groups makes chitosan a polymeric weak base. Solutions of chitosan are generally highly viscous, resembling those of natural gums.

The chitosan used herein is suitably in relatively pure form. Methods for the manufacture of pure chitosan are well known. Generally, chitin is milled into a powder and demineralized with an organic acid such as acetic acid. Proteins and lipids are then removed by treatment with a base, such as sodium hydroxide, followed by chitin deacetylation by treatment with concentrated base, such as 40 percent sodium hydroxide. The chitosan formed is washed with water until the desired pH is reached.

The properties of chitosan relate to its polyelectrolyte and polymeric carbohydrate character. Thus, it is generally insoluble in water, in alkaline solutions at pH levels above about 6.5, or in organic solvents. It generally dissolves readily in dilute solutions of organic acids such as formic, acetic, tartaric, glycolic, lactic and citric acids, and also in dilute mineral acids, except, for example, sulfuric acid. In general, the amount of acid required to dissolve chitosan is approximately stoichiometric with the amino groups. Since the pKa for the amino groups present in chitosan is between 6.0 and 7.0, they can be protonated in very dilute acids or even close to neutral conditions, rendering a cationic nature to this biopolymer. This cationic nature is the basis of many of the benefits of chitosan. Indeed, chitosan material can be considered as a linear polyelectrolyte with a high charge density which can interact with negatively charged surfaces, like proteins (e.g., by interfering with the proteinic wall construction of microorganisms, thereby acting as an antimicrobial agent and odour control agent, and/or by reacting with the proteins present in bodily fluid, like milk discharge, thereby acting as a gelifying agent for such fluid) and/or by reacting with absorbent gelling materials, namely anionic absorbent gelling materials if present in the pad.

Although chitosan material might be used as the sole fluid control material in the breast pads herein it might be used in a preferred embodiment of the present invention in combination with absorbent gelling material, to provide outstanding overall fluid absorption properties. Indeed, the use of chitosan materials together with anionic absorbent gelling materials, preferably anionic synthetic absorbent gelling materials, in breast pads provides improved absorbing performance not only towards water but especially towards electrolyte-containing fluids like lactational fluids. More generally, this combination provides enhanced fluid handling properties, i.e., not only improved absorption performance towards water and especially towards electrolytes-containing fluids, this even under pressure conditions, but also reduced wetting through (i.e., reduced fluid leakage) and reduced rewetting of the topsheet. Thus such combination participate to further enhanced comfort and protection in use.

Preferred chitosan materials for use herein have an average degree of deacetylation (D.A.) of more than 75%, preferably from 80% to about 100%, even more preferably from 90% to 100% and most preferably from 95% to about 100%. The degree of deacetylation refers to the percentage of the amine groups that are deacetylated. This characteristic is directly related to the hydrogen bonding existing in this biopolymer, affecting its structure, solubility and ultimately its reactivity. The degree of deacetylation can be determined by titration, dye adsorption, UV-VIS, IR, and NMR spectroscopy.

The degree of deacetylation will influence the cationic properties of chitosan. By increasing the degree of deacetylation the cationic character of chitosan materials will increase and thus their antimicrobial properties, their absorbing ability and gelifying ability.

Chitosan materials (i.e., chitosan and -chitosan salts, modified chitosans and cross-linked chitosans) may generally have a wide range of molecular weights. Chitosan materials with a wide range of molecular weights are suitable for use in the present invention, typically chitosan materials for use herein have a molecular weight ranging from 1 000 to 10 000 000 grams per gram moles and more preferably from 2 000 to 1 000 000. Molecular weight means weight average molecular weight. Methods for determining the weight average molecular weight of chitosan materials are known to those skilled in the art. Typical methods include for example light scattering, intrinsic viscosity and gel permeation chromatography. It is generally most convenient to express the molecular weight of a chitosan material in terms of its viscosity in a 1.0 weight percent aqueous solution at 25°C with a Brookfield viscometer. It is common to indirectly measure the viscosity of the chitosan material by measuring the viscosity of a corresponding chitosan salt, such as by using a 1.0 weight percent acetic acid aqueous solution. Chitosan materials suitable for use in the present invention will suitably have a viscosity in a 1.0 weight percent aqueous solution at 25°C of from about 1 mPa·s (1 centipoise) to about 80,000 mPa·s (80,000 centipoise), more suitably from about 30 mPa·s (30 centipoise) to about 10,000 mPa·s (10,000 centipoise), even more suitably from 50 mPa·s (50 centipoise) to about 1,000 mPa·s (1,000 centipoise) and most suitably from 100 mPa·s (100 centipoise) to about 500 mPa·s (500 centipoise).

Chitosan materials pH depends on the preparation of the chitosan materials. Preferred chitosan materials for use herein have an acidic pH, typically in the range of 4 to 6, more preferably from 4 to 5.5 and even more preferably from 4.5 to 5.5. Highly preferred pH is around pH5, which corresponds to the skin pH. By 'pH of chitosan material' it is meant herein the pH of a 1% chitosan solution (1 gram of chitosan material dissolved in 100 grams of distilled water) measured by pH-meter.

Chitosan materials with acidic pH are preferred herein as the cationic character of acidic chitosan materials will be increased and thus their antimicrobial properties, odour and fluid absorbing ability and gelifying ability. However too high acidity is detrimental to skin safety. Thus it is highly preferred herein to use chitosan materials with a pH in the range of 4.5 to 5.5, thereby delivering the best compromise between odor control and fluid handling properties on one side and skin compatibility on the other side.

Particularly suitable chitosan materials for use herein are chitosan salts, especially water soluble chitosan salts. A variety of acids can be used for forming chitosan salts. Suitable acids for use are soluble in water or partially soluble in water, are sufficiently acidic to form the ammonium salt of chitosan and yet not sufficiently acidic to cause hydrolysis of chitosan, and are present in amount sufficient to protonate the reactive sites of chitosan.

Preferred acids can be represented by the formula:

$$R\text{-}(COOH)_n$$

wherein n has a value of 1 or 2 or 3 and R represents a mono- or divalent organic radical composed of carbon, hydrogen and optionally at least one of oxygen, nitrogen and sulfur or R is simply an hydroxyl group. Preferred acids are the mono- and dicarboxylic acids composed of carbon, hydrogen, oxygen and nitrogen (also called herein after amino acids). Such acids are highly desired herein as they are biologically acceptable for use against or in proximity to the human body. Illustrative acids, in addition to those previously mentioned include, among others, citric acid, formic acid, acetic acid, N-acetylglycine, acetylsalicylic acid, fumaric acid, glycolic acid, iminodiacetic acid, itaconic acid, lactic acid, maleic acid, malic acid, nicotinic acid, 2-pyrrolidone-5-carboylic acid, salicylic acid, succinamic acid, succinic acid, ascorbic acid, aspartic acid, glutamic acid, glutaric acid, malonic acid, pyruvic acid, sulfonyldiacetic acid, benzoic acid, epoxysuccinic acid, adipic acid, thiodiacetic acid and thioglycolic acid. Any chitosan salt formed from the reaction of chitosan with any of these acids are suitable for use herein.

Examples of chitosan salts formed with an inorganic acid include, but are not limited to, chitosan hydrochloride, chitosan hydrobromide, chitosan phosphate, chitosan sulphonate, chitosan chlorosulphonate, chitosan chloroacetate and mixtures thereof. Examples of chitosan salts formed with an organic acid include, but are not limited to, chitosan formate, chitosan acetate, chitosan lactate, chitosan glycolate, chitosan malonate, chitosan epoxysuccinate, chitosan benzoate, chitosan adipate, chitosan citrate, chitosan salicylate, chitosan propionate, chitosan nitrilotriacetate, chitosan itaconate, chitosan hydroxyacetate, chitosan butyrate, chitosan isobutyrate, chitosan acrylate, and mixtures thereof. It is also suitable to form a chitosan salt using a mixture of acids including, for example, both inorganic and organic acids.

Highly preferred chitosan salts for use herein are those formed by the reaction of chitosan with an amino acid. Aminoacids are molecules containing both an acidic and amino functional group. The use of aminoacids instead of other acids, is highly preferred as those chitosan amino salts have higher skin compatibility. Indeed most of the aminoacids are naturally present on the skin. Chitosan salts of pyrrolidone carboxylic acid are effective moisturizing agents and are non irritating to skin.

Amino acids for use herein include both linear and/or cyclo amino acids. Examples of amino acids for use herein include, but are not limited to, alanine, valine, leucine, isoleucine, prolinephenylalanine, triptofane, metionine, glycine, serine, cysteine, tyrosine, asparagine, glutamine, aspartic acid, glutamic acid, lysine, arginine, istydine, hydroxyproline and the like. A particularly suitable example of cyclo amino acid is pyrrolidone carboxylic acid, which is a carboxylic acid of pyrrolidin-2-one as per following formula:

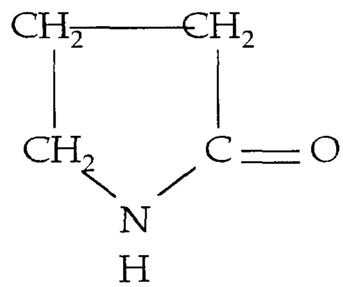

Highly preferred chitosan salts are chitosan pyroglutamate salt, which is a mixture of chitosan (a macromolecule) and pyroglutamic acid (independent monomers), chitosonium pyrrolidone carboxylate, which is the chitosan salt of 2-pyrrolidone-5-carboxylic acid.

Reference is made to WO98/07618 which describes in details processes for the preparation of such chitosan salts.

Other chitosan materials suitable for use herein include cross-linked chitosans and modified chitosans.

Crosslinking agents suitable for use in the present invention are generally watersoluble and do not substantially reduce the antimicrobial properties of chitosan. One suitable crosslinking agent is an organic compound having at least two functional groups or functionalities capable of reacting with active groups located on the chitosan materials. Examples of such active groups include, but are not limited to, carboxylic acid (-COOH), amino ($-NH_2$), or hydroxyl (-OH) groups. Examples of such suitable crosslinking agents include, but are not limited to, diamines, polyamines, diols, polyols, polycarboxylic acids, polyoxides and the like. One way to introduce a crosslinking agent with the chitosan solution is to mix the crosslinking agent with chitosan during preparation of the solution. Another suitable crosslinking agent comprises a metal ion with more than two positive charges, such as $AL^{3+}$, $Fe^{3+}$, $Ce^{3+}$, $Ce^{4+}$, $Ti^{4+}$,$Zr^{4+}$, and $Cr^{3+}$. Since the cations on chitosan possess antimicrobial properties, it is preferred herein to not use a crosslinking agent reacting to the cations, unless no alternative crosslinking agent is available.

In the embodiment herein where crosslinking agents are used, a suitable amount of crosslinking agent is from 0.001 to 30 weight percent based on the total dry weight of chitosan used to prepare the crosslinked-chitosan, more specifically from 0.02 to 20 weight percent, more specifically from 0.05 to 10 weight percent and most preferably from 0.1 to 5 weight percent.

Modified chitosans for use herein are any chitosan where the glucan chains carry pendant groups. Examples of such modified chitosans include carboxymethyl chitosan, methyl pyrrolidinone chitosan, glycol chitosan and the like. Methyl pyrrolidone chitosan is for instance described in US 5 378 472, incorporated herein by reference. Water soluble glycol chitosan and carboxymethyl chitosan are for instance described in WO87/07618, incorporated herein by reference

Particularly suitable modified chitosans for use herein include water soluble covalently bonded chitosan derivatives or ionically bonded chitosan derivatives obtained by contacting salt of chitosan with electrophilic organic reagents. Such water soluble chitosan derivatives are described in EP-A 737 692, which is herein incorporated by reference.

Suitable chitosan materials for use herein are commercially available from numerous vendors. Exemplary of a commercially available chitosan materials are those available from for example the Vanson Company. The preferred chitosan salt for use herein is chitosan pyrrolidone carboxylate (also called chitosonium pyrrolidone carboxylate) which has a degree of deacetylation of more than 85%, a water solubility of 1% (1 gram is soluble in 100 grams of distilled water at 25°C and one atmosphere), a pH of 4.5 and a viscosity between 100-300 cps. Chitosan pyrrolidone carboxylate is commercially available under the name Kytamer® PC from Amerchol Corporation.

Typically, the breast pads might comprise chitosan material or a mixture thereof at a level of from 0.1 $gm^{-2}$ to 300 $gm^{-2}$, preferably from 0.5 to 100 $gm^{-2}$, more preferably from 3 $gm^{-2}$ to 50 $gm^{-2}$ and most preferably from 4 $gm^{-2}$ to 30$gm^{-2}$

Shape and geometry of the breast pad

Advantageously the breast pads herein because they are made of conformable material can be shaped before or during use, from a generally plane configuration into a configuration to fit the woman breast by elastic and/or plastic deformation. The breast pads might be circular (including circle to ellipsoidal forms). In a preferred execution the pad has a geometry so that at least more than 50% of the surface area of the pad is disposed beneath the breast nipple. With such a pad configuration (see Figures 3 and 4) it is possible to achieve a more discreet and better fit to the breast, and thereby ensure a high degree of satisfaction for the nursing woman as she can act more freely as if she was wearing conventional products. Advantageously when at least more than 50% of the projected area of the absorbent core is disposed beneath the breast nipple the risk of leakage is seen to be diminished considerably. Accordingly in such an embodiment the topsheet, backsheet and absorbent core constituting the pad are substantially triangular in shape. The substantially triangular type shape is meant to cover all geometries from a simple triangle to pads having flaps extending from each of the three corners.

In the preferred embodiment herein the topsheet, backsheet and/or absorbent core are shaped into a configuration to fit the woman breast before or during the manufacturing process of the pad. With this embodiment, the user will avoid the inconvenience of having to shape the pads, and it is possible especially when choosing a radius of curvature for the breast pad less than the natural radius of curvature of the breast to ensure that the pressure from the breast pad will mainly lie outside of the nipple area, at the same time as the breast pad lies in abutment with the breast with an anatomical curvature. A further way of avoiding the nipple from being subjected to pressure is when the central portion of the pad according to the invention includes a depression adapted to receive the breast nipple.

In a highly preferred embodiment the breast pad is shaped in 3D configuration with a given radius of curvature, this radius being either uniform in all directions (from nipple area to outer edges of the breast pad) or non uniform, e. g., different radius of curvatures can be envisaged in different directions of the breast pad. Preferably the radius of curvatures are different in the horizontal and vertical planes of the breast pad so as to better adapt to the various anatomies of women breast.

The vaulted or arched triradial and preferably asymmetrical shape of the breast pad is considerably better with regard to stay in place. Further the use of the material as described herein in combination with such a shape provides a softer and more pleasant breast pad capable of following the movements of the breast in a more natural manner.

It is however, particularly beneficial if the absorbent core is provided with circular pre-scoring lines directed towards the center (nipple area of the pad) such that the garment facing surface of the absorbent core on the periphery/outer edge more readily fold towards the center of the pad (scoring would be on the garment facing side of the absorbent core). If the breast pad is made from a continues band of a laminate of all material then scoring can also be done on the complete product rather than just the core. This would further help the conformability of the breast pads

Breast pad construction

In one embodiment the absorbent core of the breast pad extends to the periphery/outer edges of the breast pads, namely the topsheet and backsheet.

In the preferred embodiment the topsheet and the backsheet have dimensions generally larger than the absorbent core so that they extend beyond the edges/periphery of the absorbent core, where they are associated together in a suitable manner.

Adjacent layers of the breast pads might be joined by any suitable manner, including any attachments means known to those skilled in the art like an uniform continuous layer of adhesive, a patterned layer of adhesive or an array of separate lines or spots of adhesives.

For example a welding system or hot melt adhesive might be used to seal the topsheet and the backsheet together in the area where they extend beyond the periphery of the absorbent core. Such welding system might be typically used herein especially in embodiment herein wherein the backsheet is made of a liquid impermeable water vapor permeable composite structure comprising a hydrophilic thermoplastic film and a support layer as described herein before and the topsheet is made of an apertured formed film. Such hot met adhesive might be used in the embodiment herein wherein the backsheet is a dual layer of thermoplastic film and nonwoven layer. The adhesive might be used (hot melt) to seal together the topsheet and the backsheet in the area extending beyond the periphery of the absorbent core but also at least partially the different layers of such a backsheet. In a particularly preferred embodiment of the present invention it is preferred not to cover the full surface of such adjacent layers of the backsheet like the first and second layer of the backsheet, typically applying the adhesive only around the circumference of the adjacent layers without the central area of that layers (i.e. nipple area). The surface free of adhesive in the central area of the breast pad helps to avoid the strike through of the liquid from the core to the bra. Moreover less adhesive/glue helps to slide (slippage) of the layers during the deformation (thereby resulting in less wringles). Also in the preferred

execution herein wherein the topsheet is made of two layers, the outer layer facing the wearer surface and the underlying layer facing the absorbent core, might be bond together by a continuous pattern of a small amount of hot melt glue (typically spiral spray, but any other conventional type of application is suitable for use herein) which creates an high capillarity effect for fluids to move from topsheet to the core (liquid transfer). Alternatively radial embossing or other kinds of embossing between the outer layer of the topsheet and secondary topsheet may give the same intimate contact.

Optional non slip properties

According to the present invention the wearer facing surface of the pad and/or the garment facing surface of the pad can be provided with non-slip properties.

In its broadest aspect the present invention also encompasses a breathable breast pad (typically a breast pad having a water vapor permeability higher than 100(g)/(m2/24hrs) as defined herein and/or an air permeability higher than 50l/m2/s (as defined herein) ), the breast pad having a wearer facing surface and a garment facing surface, wherein at least one of said outer surfaces has a coefficient of friction greater than 1.

The term 'non-slip' is generally used herein to describe breast pad according to the present invention having at least one outer surface of the breast pad, i.e., the wearer facing surface and/or the garment facing surface, typically the outer surface of the topsheet and/or outer surface of the backsheet (in other words, the surfaces not facing the absorbent core) which has been modified/treated so as to result in substantially reduced slippage when used in contact against human skin and/or bra/garment. The static coefficient of friction of this surface is generally more than 1.0, preferably more than 1.2, even more preferably more than 1.4 and most preferably more than 1.6.

The coefficient of friction of the non-slip surface can be evaluated according to ASTM D 1894, Standard test Method for static and kinetic coefficients of friction of plastic film and sheeting, using a tensile tester, Instron model 6021 with the 6000 series control console with the "Peel, Tear, Block and Friction test" Software Program code 6100-014 (for selecting the test friction function). Test parameters are: Initial length 2mm, end point 100mm, test speed 150mm/min. The test is performed using the moving sled-stationary metallic plane (ASTM method of assembling C). Metallic plane (also called 'plate') having a dimension of 515*152*9 mm.

According to the present invention all measurements were made using a 6.25cm by 6.25cm square sled of 200g weight. The coefficient of friction referred to herein is the result of friction measurements made according to this ASTM test method between the outer surface of the breast pad and a metallic plane covered by a reference material that is white 100% cotton weave style 429-w cut to 76mm*460mm supplied by testex 53947 Nettersheim Germany. For each test measurement the cotton will be replaced with a new one. The cotton is clamped onto the metallic plane only on one end opposite to the traction (pull) of the sled.

The non-slip materials suitable for used according to the present invention may be any material including any latex or hot melt coating that has sufficient skid-resistance properties to be able to match the coefficient of friction defined herein when applied on an outer surface of the breast pad.

The non-slip materials suitable to use herein include, but are not limited to, materials of the following groups of materials:

- Ethylene vinyl acetate copolymers that can be applied as a hot melt or as a water based coating. Preferred materials have at least 28% vinyl acetate,
- Polyvinyl acetate such as those typically used in water based emulsions
- Styrene-butadiene - applied in an emulsion or as a hot melt,
- Cellulose acetate butyrate - normally hot melt coatings,
- Ethyl cellulose - normally blended with a plasticizer and a resin and applied as hot melt,
- Acrylics - normally emulsion system that are not blended,
- Elastomers,
- Synthetic rubber hot melt - Kraton® block copolymers having elastomeric and styrenic blocks, rubber, resin, plasticizer blends,
- other hot melts -polyethylenes (alone or blended), polyamides.

Preferred non-slip materials for use herein are ethylene-vinyl acetate copolymers, acrylic terpolymers of methacrylic acids, acrylic copolymers, ethylene-vinyl acetate/resin latex emulsions, ethylene-vinyl acetate hot-melt adhesives, synthetic rubber (block copolymers with elastomeric and styrenic components) hot melt adhesives and polyvinyl acetate/resin emulsions. Such materials are available from H.B. Fuller Company, E.1. Dupont and Findley Adhesives, among others.

In a particular embodiment, the non-slip materials for use herein are those delivering some water vapor permeability through their thickness. Indeed some of the non-slip materials for use herein might have a water vapor transfer rate of at least 100 g/$m^2$·24h, more preferably at least 300 g/$m^2$·24h, and most preferably at least 500 g/$m^2$·24h, when used in the form of continuous layer of a thickness of at least 0.5 μm.

Surprisingly by using such coating materials in continuous application on the outer surface of the backsheet of a breast pad the water vapor permeability of the backsheet of the breast pad is reduced only to a minimum extend or even not reduced at all. In other words, non-slip properties might be provided without impairing on the breathability properties of the backsheet. The preferred non-slip materials for use herein are hydrophilic non-slip materials per se or applied in a hydrophilic form (e.g., water-based solution/dispersion or emulsion) which have the ability to transfer substantial amounts of water vapor through their thickness by absorbing water on one side where the water vapor concentration is higher, and desorbing or evaporating it on the opposite side of their thickness where the water vapor concentration is lower.

Particularly suitable non-slip water vapor permeable materials for use herein include water permeable polymeric resins such as elastomers containing urethane bonds, which are permeable to passage of water vapor through their thickness. Such elastomers containing urethane bonds include silicones (e.g., heat-cured silicones, condensation-cured silicones and RTV silicones) polyurethanes. A preferred elastomer is RTV 863 from GE Silicones, Inc., Waterford, NY.

Other particularly suitable non-slip water vapor permeable materials include copolymers of alkyl ester of acrylic acid with comonomers. Such alkyl ester of acrylic acid copolymer based materials are particularly suitable as they combine the benefits of easy processability, delivering soft hand or feel, while being odor free and color free (in contrast to for example elastomeric polymers which usually have a non desirable odor and impair a yellow/brunish color to the surface/layer treated therewith which may not be acceptable by the user as not contributing to the desire of discretion of the product). Advantageously such alkyl ester of acrylic acid copolymer based materials confer non-slip properties not only in dry conditions but also in wet conditions (typically upon accidental wet/soil through occurrence). Also such copolymer based materials have the ability to cure at room temperature. In other words crosslinking of the copolymer based material will not be affected during normal usage at room temperature, thereby contributing to increased non-slip durability in normal usage conditions. Additionally such copolymer based materials are non tacky at room temperatures, thereby providing excellent non-slip properties without stickiness. Indeed such polymer based materials have a Glass Transition Temperature (Tg) which is below -40 degree Celsius. Below such temperature the polymer based materials are stiff and become softer above. At usage temperatures they have enhanced softness.

Such copolymer based materials might contain from 55% to 90% by weight of an alkyl ester of acrylic acid, wherein the alkyl group may contain from 1 to 8 carbon atoms, from 5% to 40% by weight of acrylonitrile and from 0.5% to 10% by weight of a crosslinking comonomer, the total of course being 100%. Typical crosslinking comonomer for use herein include N-methylol acrylamide, N-methylol methacrylamide, glycidyl acrylate, glycidyl methacrylate and the like.

Such copolymer might be prepared by any suitable polymerization method. Usually the polymerization is carried out in an organic solvent medium by heating the mixture of comonomers at the reflux temperature of the solvent, thus insuring a constant temperature and dissipation of the heat of reaction in evaporating the solvent. Solvent soluble free radical initiating catalysts of the peroxide or azo type are utilized in such polymerisation. If the resulting polymer is insoluble in the solvent, it can be filtered, washed and thereafter dissolved in a solvent in which it is soluble. The copolymers can be utilized in the form of the lacquers wherein they were prepared, or these lacquers, in turn, may be converted to aqueous emulsions prior to their application to the surface to treat. Thus, lacquers of these copolymers may be readily emulsified by adding an aqueous solution of an emulsifier, such as polyvinyl alcohol, morpholine-oleic acid mixtures and the like, to the lacquers while the lacquers are being vigorously agitated. The organic solvent is then removed from the mixture by flash distillation or any other suitable method. The polymerization may also be carried out by dispersing the comonomers as fine droplets in a large volume of water through the use of emulsifying agents like surfactants. Then the polymerization is effected in the presence of water soluble catalysts, such as, for example, the persulfates. At any rate the solid contain of the copolymer may range from 30% to 65% by weight.

In addition to the copolymers described previously, the non-slip copolymer based material may also include such other ingredients as inert fillers, pigments, catalytic agents, antioxidants, ultra violet stabilisers, diluents, thermosetting resins, plasticizers and the like if desired.

Example of commercially available alkyl ester of acrylic acid based copolymer is Nacrylic X4445® from National Starch. Such copolymers are fully described in US 3 336 149, which is incorporated herein by reference.

The non-slip material might be applied by any conventional coating method known to those skilled in the art including spraying, screen or flock printing or by rod coating or gravure printing processes.

Typically the non-slip material like an alkyl ester of acrylic acid based copolymer commercially available from National Starch under trade name Nacrylic X4445®, in a dispersed hydrophilic emulsion form (the polymer is a surfactant stabilized polymer-containing water based emulsion) is applied as a surface coating to the surface/layer to be treated, preferably nonwoven, using preferably an airless spray technology, which utilizes hydraulic pressure instead of air to atomise and spray. This can be undertaken with the dispersion at ambient temperature or by pre-heating up to 90 degrees centigrade. The dispersion can be sprayed at a non volatile content as supplied from the manufacturer (typically 35 - 60% solid content) or upon dilution with water to achieve the required dry binder add on to the support layer like nonwoven. Drying and curing of the polymer dispersion is achieved by the application of heat, typically by passing the bonded web through an Industrial scale oven until the desired end use characteristics have been attained.

The non-slip coating materials might be applied onto the outer surface of the breast pad in any conventional way, being continuous or discontinuous. By 'discontinuous' application it is meant herein that portions of the outer surface of the breast pad are left uncoated by the non-slip material. These uncoated portions therefore may contribute to maintain high water vapor permeability and/or high air permeability. In the embodiment herein wherein a non-slip material with the properties of being water vapor permeable is used to treat a water vapor permeable backsheet the application pattern used only slightly influence the water vapor permeability of such a breathable backsheet.

The non-slip coating materials might be applied in the form of small dots or domes (in circular forms, polygonal forms, square, pentagonal and the like) at spaced intervals or may be intersecting lines in various patterns such a grid pattern, straight rows, oblique rows or any other arrangements according to the intended application. The non-slip material may be applied in a discontinuous pattern extending across only a portion of the surface area of the total surface of the outer surface of the breast pad, preferably having a size and distribution appropriate to cover between at least 1% and 98% of the total surface area of the outer surface of the breast pad (i.e., the wearer facing surface and/or garment facing surface of the breast pad), typically topsheet and/or backsheet to which it is applied to. More preferably the non-slip material covers about 3% to 85% of the total surface area of backsheet and/or topsheet of the breast pad and more preferably between 10% and 70%.

In one embodiment herein wherein a discontinuous pattern of non-slip material is applied onto an outer surface of the breast pad, the pattern selected for the application of the non-slip material may intentionally be directionally asymmetric in order to provide for different coefficients of friction in different directions. This condition may be particularly desirable for certain applications which might benefit from increased resistance to slippage in one direction while allowing a greater amount of slippage in another direction.

Water Vapor Permeability Test

The Vapor permeability test is utilized to quantify the vapor transmission properties of breathable breast pads.

Basic Principle of the Methods

The basic principle of the test is to quantify the extent of water vapor transmission of a breast pad. The test method that is applied is a standard one, namely ASTM E 96 - 80, Procedure B - Water Method at 23°C. The test is performed in a stable temperature/humidity laboratory maintained at a temperature of 23° C at 50% RH for a period of 24 hours.

The vapor permeability value is determined as the weight loss divided by the open area of the sample holder and quoted per day.

$$\text{i.e. Vapor Permeability} = \text{Weight Loss (g)} / (\text{m}^2 / 24\ \text{hrs})$$

Air Permeability test

The air permeability test is utilized to assess the ability of a breast pad to circulation/exchange air.

Basic Principle of the Methods

The basic principle of the test is to evaluate the resistance of a breast pad to the passage of air. In this test, the volume (or amount) of air that flows through a breast pad of given dimensions under standard conditions (of 23°C /50% RH) is measured. The instrument utilized for the test is: Air Permeabilimeter FX 3300 manufactured by TexTest AG Switzerland.

Samples should be allowed to equilibrate in the test environment for at least 4 hrs prior to commencement of the measurement. The breast pad (having dimensions exceeding 5 $cm^2$ the dimensions of the measurement head) is placed with the topsheet up on the device as instructed by the manufacturer. An aspiration pump set to generate a pressure of 1210 Pa that sucks air through the pad structure. The device measures the volume of air flow and the pressure drop across the orifices that contains the pad and measurement head. Finally the device generates a value of air permeability in the units of liters/m2/s.

Caliper Measurement

The average caliper of the breast pad is determined. The caliper at representative points (at least 5) of the pad is measured to determine an average value by using a micrometer gauge from Lorentzen & Wettre Kista Sweden cod 51D20 with a foot of 10cm2 and pressure of 20g/cm2.

Conformability test method

The stiffness of the breast pad is measured according to a modified ASTM method D4032-94, the procedure being performed as follows. The circular bend procedure is a simultaneous multi-directional deformation of a material in which one face of a specimen becomes concave and the other face becomes convex. The circular bend procedure gives a force value related to resistance to bending, simultaneously averaging stiffness in all directions. The difference of the modified ASTM D4032-94 method used according to the present invention to ASTM D4032-94 are in the orifice geometry, shape and size of the plunger and specimen. The apparatus is mounted on a tensile tester by Instron model 6021 with the 6000 series control console with the "General tension-compression test" Software Program code 6100-201/A (selecting the test tension function).

1) Apparatus:

The apparatus used for the circular bend procedure is a circular bend stiffness tester having the following parts:

A plexiglass plate platform which is 100 * 100* 2 millimeters having an 30 millimeter diameter orifice. The lap edge of the orifice should be at a 90 degree angle to a depth of 4.8 millimeters.

A plunger having a diameter of 15 millimeters, the plunger being mounted concentric with the orifice, 7.5 clearance on all sides. The bottom of the plunger should be set at 3 mm above the top of the orifice plate. From this position, the downward stroke length is 57 mm.

A force measurement gauge and more specifically an Instron inverted compression load cell. The load cell has load range of from 0 to 1000 grams

The apparatus is mounted on a tensile tester by Instron model 6021 with the 6000 series control console with the "General tension-compression test" Software Program code 6100-201/A (selecting the test tension function .

2) Number and preparation of specimens

In order to perform the procedure for this test five breast pads are necessary.
Each pad is cut with an hydraulic cut to form a circle with a diameter of 49mm, then the resulting specimens are carefully centered on the orifice plate with the topsheet up. The specimens should be handled carefully to avoid misplacement of the layers. The specimens should not be folded or bent by the test person, and the handling of the specimens must be kept to a minimum and to the edges to avoid affecting stiffness properties. Then the test is started, the plunger push the specimen through the orifice. If the specimen does not move uniformly (i.e. the edge is not aligned and parallel with the plate) the value is rejected and a new specimen is tested instead.

3) Procedure

The procedure for the circular bend procedure is as follows. The specimens are conditioned by leaving them in a room which is 23+2C and 50% +- 2% relative humidity for a period of 24hrs.The test plate is leveled at eye level. The plunger speed is set at 100 mm per minute. A specimen is centered on the orifice platform below the plunger such that the wearer surface of the specimen is facing the plunger and the garment surface of the specimen is facing the platform. The indicator zero is checked and adjusted, if necessary. The plunger is actuated. Touching the specimen

during the test is to be avoided. The maximum force reading to the nearest gram is recorded. The above steps are repeated until all five of the identical specimens have been tested.

4) Calculation

Average the individual specimen readings and round to the nearest gage increment.

Tensile strength test method under wet condition

The tensile strength of the absorbent core of the breast pads herein is measured according to modified ASTM D 5034-95, as described herein. Tensile strength is defined as the amount of force required to tear a test sample/ specimen until the sample/specimen breaks in two distinguished parts.

1) Apparatus

- An E-constant-rate-of extension (CRE) tensile testing machine, namely Instron model 6021 with the 6000 series control console with the "General tension-compression test" Software Program code 6100-201/A (selecting the test tension function) is used
  Test parameters: test speed 300mm/min.
- one inch cutter (25.4 mm)

2) Test fluid

0.9% NaCl water solution

3) Procedure

In order to perform the procedure for this test five breast pads are necessary.
The test method used derives from ASTM (American Standard Test Method) Designation: D 5034 - 95 (Standard Test Method for Breaking Strength and Elongation of Textile Fabrics (Grab Test)), and is performed as follows:
Each pad is placed in a 1 inch cutter and one test specimen per pad is cut, the resulting specimen having a wide of one inch (25.4 mm), the length of the specimen corresponding to dimension of the breast pad to be tested. The length of the specimen is not critical provided it is long enough to be maintained between the clamps during the test. The long dimension is the one parallel to the direction of testing and force application.
The operation has to be repeated in MD (machine direction) and also CD (cross direction) direction. According to the present invention it suffices that the tensile strength in at least one of these directions is of at least 550g/25.4mm, when measured under wet condition.
To identify MD and CD direction, unless otherwise specified, a first set of specimens are cut with the long dimension parallel to the warp direction of the topsheet and a second set of specimens are cut cross to the warp direction. If not possible to identify the warp direction, a first set of specimens are cut in one direction (first direction) and a second set are cut cross to the first direction. The specimens are collected and marked with "MD" or "CD" letters, in order to identify them or alternatively they are marked with "set parallel to warp direction or first direction" or "set cross to the warp direction or cross to the first direction" in order to identify them.
Then the outer layers (typically topsheet and backsheet) are separated from the absorbent core which represents the specimens to be tested. The specimens are conditioned by leaving them in a room at 23° +/- 2°C and 50% +/- 2% relative humidity for a period of 24hrs. Before being tested the specimens are wetted by being placed in a container wherein 4 ml of test fluid is added drop by drop onto the specimens, the specimens are left 5 minutes in this container before being tested on the tensile machine .
The tensile machine is calibrated and zero according to manufacturer's instruction, a load cell is used so that tensile result will be between 25% and 75% of the capacity of the load cell, the specimens are placed vertically between the clamps of the machine with the long dimension parallel to the direction of testing and force application and the test is started.
The air pressure inlet is opened, the dynamometer is switched on, apposite test program is loaded. It is to be ensured that the working area is safe, that the emergency stop works and all attachments are correct. During the test

the dynamometer should not be operated or touched. This to avoid wrong values, but also for safety reasons. Never change the specimens or perform tests while clamps are moving. The dynamometer is set up to a test speed of 300 mm/min. The distance between the clamps should be 75 mm (gage length) so that positioning of the specimens is comfortable.

4) Calculation

Average the individual specimen readings of the breaking point of the specimen

Absorption test

The absorption capacity of the breast pad according to the present invention is determined by the Absorptive capacity test described herein after (modified ASTM D 1117-80).

The present test method differs from ASTM D1117-80 in that breast pads are tested, wherein said pads are put floating on the surface of the test fluid without usage of screen, they stay on the test fluid for 25 minutes, then they are hold on a plexiglass slope with a loading on them to drain the excess of test fluid.

1) Measuring principle

This test measures the weight of a test fluid which is retained in a breast pad after it is floated on the test fluid for 25 minutes and after applying a static pressure of 17 g/cm2 on the breast pad for 2 minutes.

2) Instruments and testing aids

- Basin 20X20X2cm
- Digital balance accurate to +/-0.01 g
- Timer control (stop watch) accurate to 0.1 second (CASIO HS-10W)
- Plexiglas plate and basin (at least 2 cm deep), at least 25*25 cm
- Plexiglas Slope with 15° angle to the horizontal (same dimension than the pad to be tested)
- Weight of 1700g with base dimension 10X10cm
  (equivalent to 17g/cm2) with a foam base covered with plastic film
- A stand with an extendable arm to fix the weight on the slope and stop it from sliding

3) Test fluid

Saline solution (0.9% NaCl in water)

4) Procedure

The breast pad (overall dimension 93 cm2, caliper 2.5 mm) is pre-weighted using the digital balance and the initial dry weight to an accuracy of +/- 0.01 g is recorded.

The basin is partially filled with the test fluid. It is ensured that the depth of the test fluid in the basin throughout the test is maintained higher than 5 mm.

The breast pad is put in the basin face down (i.e. topsheet down) for 25 minutes.

The breast pad is gently removed from the basin by holding it from its edges. It is left to drip for 2 seconds in a vertical position before being placed face down on the slope.

The weight is placed gently on the breast pad for 2 minutes (+- 5 sec.) and the arm of the stand is used to fix the weight, thereby avoiding sliding.

The weight is removed after 2 minutes, the breast pad is hold from an edge portion and left to drip in a vertical position for 2 seconds, then it is reweighted and the wet weight is recorded to an accuracy of +- 0.01g.

The following precautions should be followed during the test to ensure consistent accuracy of the results:

The breast pad should be kept flat without bending or twisting in all steps of the test.

The breast pad should be held gently at the top while the load is applied to prevent it from sliding. Applying the load should be done carefully and gradually starting from the top of the test sample.

The slope and base of the weight are to be dry before placement of the breast pad. Also the balance tray should be dry before weighting the wet pad.

5) Evaluation

Absorption Capacity (g)= wet weight (g) - the initial dry weight (g)

The total capacity is given by the Absorptive capacity following the test described above
The absorption capacity can be reported in grams per square cm of the breast pad tested as well as in grams per gram of breast pad tested.
The present invention will be illustrated by the following examples

Example 1

A triangular shape breast pad 10 (caliper of 2.5 mm) is provided in Figures 3 and 4 and comprises (as seen in Figure 4):

- a topsheet 20, namely a spunbonded, surfactant treated polyethylene nonwoven commercially available from BBA (Linotec, Aschersleben, Germany) under material code: T27AXC (surfactant type: Silicone based, hydrophilic, non-permanent at concentration of 0.2% g/g, applied by spray-process)
- a secondary topsheet 30, namely an apertured hydroentangled nonwoven (67% viscose, 33% Polyester) supplied by J. Suominen Oy (Finland) under material code: Fibrella 2200/50
- a spiral spray layer of adhesive (not shown) (H2031® from ATO Findley) between topsheet and secondary topsheet
- an absorbent core 40, namely a needle punched of 60% viscose and 40% Fiberdri (viscose: Courtaulds' fibers 1.5 d x 40mm type 18453, Fiberdri: Camelot' superabsorbent fiber 9d x 50mm type 908) commercially available from Texel Inc (Canada),
- spiral spray layer of adhesive (not shown) (H2031® from ATO Findley) between the secondary topsheet and absorbent core,
- a backsheet comprising a thermoplastic film 50 (supplied by H.B. Fuller) coated to a thermalbonded 100% Polypropylene nonwoven 60 supplied by liesstoffwerk Chr. H. Sandler GmBh& Co.KG under material code: Sawabond 4124®.

The nonwoven is positioned on the outer side of the breast pad 10, the garment facing side 70. The topsheet and the backsheet are sealed together into a peripheral flange (area extending beyond the periphery of the absorbent core) by welding (not shown).
The thermoplastic film used in water vapour permeable composite structure as the backsheet is obtained by compounded a polyether-amide block copolymer available from Elf Atochem (France) under the trade name Pebax 2533® with acetyltributyl citrate available from Reilly Chemicals under trade name Citroflex A4®, resins available from Hercules under code name Res A-2690 and Res A-2691 and Irganox 1010® and PS800® (antioxidant agent) available from Ciba-Geigy.
The final thermoplastic film composition in percent by weight is as follows:

Resin available from Hercules Inc. under code name Res A-2690 (14.85%)

Resin available from Hercules Inc. under code name Res A-2691 (14.85%)

Both these resins are described in European patent application No.00116284.1

Acetyltributyl citrate Citroflex A4® from Reilly Chemicals (25%)

Polyether Block Amides Pebax 2533® from Elf Atochem (45%)

Irganox 1010® (0.15%) from Ciba Geigy

Irganox PS 800® (0.15%) from Ciba Geigy

The blend was melt coated at 175°C to obtain a film having a thickness of about 50µm. The film was then laminated directly onto nonwoven thermalbonded 100% Polypropylene supplied by liesstoffwerk Chr. H. Sandier Gm-

Bh& Co.KG under material code: Sawabond 4124®.

Example 2

A breast pad as described in Example 1 is made except that the nonwoven backsheet is provided with non-slip properties. Accordingly the nonwoven is treated with self-crosslinking acrylic copolymer emulsion commercially available from National Starch under material code: Nacrylic X4445®.

Indeed Nacrylic X4445® (commercially available at a solid content of 45%) is diluted with water (90g of polymer at 45% of solid content with 10g of water). This resulting solution is sprayed onto the nonwoven already coated to the thermoplastic film before incorporating the backsheet in the breast pad. The spray operation is conducted at room temperature (23°C +-2°C) by spraying 21.73g/m2 (wet polymer) which corresponds to 8.8g/m2 (dry polymer) after drying at room temperature.

Example 3

A circular breast pad (diameter 10.5 cm and caliper of 2.5 mm) has been made as follow and comprises :

- a topsheet , namely a spunbonded, surfactant treated polyethylene nonwoven commercially available from BBA (Linotec, Aschersleben, Germany) under material code: T27AXC (surfactant type: Silicone based, hydrophilic, non-permanent at concentration of 0.2% g/g, applied by spray-process)
- a secondary topsheet , namely an apertured hydroentangled nonwoven (67% viscose, 33% Polyester) supplied by J. Suominen Oy (Finland) under material code: Fibrella 2200/50
- a spiral spray layer of adhesive (H2031® from ATO Findley) between topsheet and secondary topsheet
- an absorbent core , namely a needle punched of 60% viscose and 40% Fiberdri (viscose: Courtaulds' fibers 1.5 d x 40mm type 18453, Fiberdri: Camelot' superabsorbent fiber 9d x 50mm type 908) commercially available from Texel Inc (Canada),
- spiral spray layer of adhesive (H2031® from ATO Findley) between the secondary topsheet and absorbent core,
- a backsheet comprising a thermoplastic film (supplied by H.B. Fuller) coated to a thermalbonded 100% Polypropylene nonwoven supplied by liesstoffwerk Chr. H. Sandler GmBh& Co.KG under material code: Sawabond 4124®.

The nonwoven is positioned on the outer side of the breast pad, the garment facing side. The topsheet and the backsheet are sealed together into a peripheral flange (area extending beyond the periphery of the absorbent core) by welding (not shown).

The thermoplastic film used is the same as those described herein in Example 1 above.

Example 4

A breast pad as described in Example 3 is made except that the nonwoven backsheet is provided with non-slip properties. Accordingly the nonwoven is treated with self-crosslinking acrylic copolymer emulsion commercially available from National Starch under material code: Nacrylic X4445®.

Indeed Nacrylic X4445® (commercially available at a solid content of 45%) is diluted with water (90g of polymer at 45% of solid content with 10g of water). This resulting solution is sprayed onto the nonwoven already coated to the thermoplastic film before incorporating the backsheet in the breast pad. The spray operation is conducted at room temperature (23°C +-2°C) by spraying 21.73g/m2 (wet polymer) which corresponds to 8.8g/m2 (dry polymer) after drying at room temperature.

Example 5

A circular breast 1 (diameter 10.5 cm and caliper of 2.8) is provided in a shaped configuration in Figures 1 and 2 and comprises (as seen in Figure 1-cross section of pad construction):

- a topsheet 2, namely a spunbonded, surfactant treated polyethylene nonwoven commercially available from BBA (Linotec, Aschersleben, Germany) under material code: T27AXC (surfactant type: Silicone based, hydrophilic, non-permanent at concentration of 0.2% g/g, applied by spray-process)
- a secondary topsheet 3, namely an apertured hydroentangled nonwoven (67% viscose, 33% Polyester) supplied by J. Suominen Oy (Finland) under material code: Fibrella 2200/50
- an absorbent core 4, namely a needle punched of 60% viscose and 40% Fiberdri (viscose: Courtaulds' fibers 1.5 d x 40mm type 18453, Fiberdri: Camelot' superabsorbent fiber 9d x 50mm type 908) commercially available from

Texel Inc (Canada),

- spiral layer of adhesive (not shown) (H2031® from ATO Findley ) between the topsheet and secondary topsheet as well as between the secondary topsheet and the absorbent core,
- a first backsheet layer 5 made of liquid impervious polymeric film having apertures available from Tredegar Film Products B.V., material available under supplier code 'slanted cones'
- a second backsheet layer 6 made of nonwoven thermalbonded 100% Polypropylene supplied by liesstoffwerk Chr. H. Sandier GmBh& Co.KG under material code: Sawabond 4124®.
- a spiral spray layer of adhesive (not shown) (H2031® from ATO Findley ) between the absorbent core and the first backsheet layer
- the topsheet 2 and the backsheet 6 are sealed together into a peripheral flange (area extending beyond the periphery of the absorbent core) by the same hot melt adhesive positioned between the absorbent core and the first layer backsheet layer.

Example 6

A breast pad as described in Example 5 is made except that the nonwoven backsheet is provided with non-slip properties. Accordingly the nonwoven is treated with self-crosslinking acrylic copolymer emulsion commercially available from National Starch under material code: Nacrylic X4445®.

Indeed Nacrylic X4445® (commercially available at a solid content of 45%) is diluted with water (90g of polymer at 45% of solid content with 10g of water). This resulting solution is sprayed onto the nonwoven backsheet before it is incorporated into the breast pad. The spray operation is conducted at room temperature (23°C +-2°C) by spraying 21.73g/m2 (wet polymer) which corresponds to 8.8g/m2 (dry polymer) after drying at room temperature.

Table

Below table is a comparison table between breast pads according to the present invention namely those exemplified in Examples 4 and 6 described herein versus commercially available competitive breast pads.

Different parameters are compared. This table is indicative. It is understood herein that the scope of the present invention is not limited to the breast pads exemplified herein nor to the parameters compared in this table.

This table compares breast pads illustrated herein before in Examples 4 and 6 to breast pads commercially available namely (1) from Johnson & Johnson under the trade name "Healthflow Ultra Nursing Pad" Safeway Denver Co, (2) Gerber under the trade name "Super Absorbent Nursing Pad" on the US market and (3) under the name 'Pigeon breast pad' by Santo Health Care Products for their comformability properties (stiffness under modified ASTM method D4032-94 described herein before), water vapor permeability (expressed in g/$m^2$/24 hrs, as per ASTM E 96 - 80 test method mentioned herein before), integrity properties of the breast pads (tensile strength under wet conditions of the absorbent core as per modified ASTM D 5034-95), absorption capacity (as per modified ASTM D 1117-80 described herein before) caliper and coefficient of friction (as per ASTM D 1894).

(1) Breast pad available from Johnson & Johnson is made of a spunlaced nonwoven and aifelt absorbent core and a nonwoven backsheet, this pad is free of super absorbent gelling material (AGM).

(2) Breast pad available from Gerber is made of an airfelt with synthetic fibers as an absorbent core, sandwiched between a thermalbonded nonwoven topsheet and a nonwoven backsheet, this pad is free of super absorbent gelling material (AGM).

(3) Pigeon breast pad is made of an absorbent core made of airfelt with AGM powder sandwiched between wetlaid nonwoven layers, an air through biconponent nonwoven topsheet and a non breathable polyethylene/wetlaid nonwoven and fastening adhesive and release paper.

| | | Breast pad of Example 4 | Breast pad of Example 6 | Healthflo w Ultra Nursing Pad-J&J | Super Absorbent Nursing Pad Gerber | Pigeon breast pad |
|---|---|---|---|---|---|---|
| Stiffness | g | 308 | 250 | 1940 | 627 | 325 |
| WVTR | g/($m^2$/24 hrs) | 400 | 592 | 896 | 1070 | 46 |

(continued)

| | | Breast pad of Example 4 | Breast pad of Example 6 | Healthflo w Ultra Nursing Pad-J&J | Super Absorbent Nursing Pad Gerber | Pigeon breast pad |
|---|---|---|---|---|---|---|
| Caliper | mm | 2.5 | 2.8 | 5.9 | 3.5 | 2.3 |
| Coefficient of friction | - | 1.9 | 1.9 | 0.7 | 1.1 | bra fastening adhesive |
| Tensile Strength with 0.9% NaCl | g/25.4mm | 2100 | 2100 | 51 | 535 | 67 |
| Capacity with 0.9% Nacl | g/cm$^2$ | 0.31 | 0.31 | 0.38 | 0.30 | 0.53 |
| Capacity with 0.9% NaCl | g | 26.7 | 26.7 | 30.5 | 24.5 | 65.8 |

**Claims**

1. A disposable thin breathable breast pad having a wearer facing surface and a garment facing surface, said breast pad having a water vapor permeability of more than 100 (g) / (m$^2$/24hrs) and a stiffness of less than 600 grams.

2. A pad according to claim 1, having a water vapor permeability of more than 100 (g) / (m$^2$/24hrs), more preferably of more than 200 (g) / (m$^2$/24 hrs), and most preferably higher than 300 (g) / (m$^2$/ 24 hrs).

3. A pad according to any of the preceding claims having a stiffness of less than 500 grams, more preferably less than 400 grams, and most preferably of less than 350 grams.

4. A pad according to any of the preceding claims having a fluid absorption capacity of at least 0.05 grams per cm$^2$, preferably of at least 0.10 grams per cm$^2$, preferably from 0.15 to 1.50, more preferably from 0.20 to 0.80 and most preferably from 0.25 to 0.6 grams per cm$^2$.

5. A pad according to any of the preceding claims having a thickness of less than 5.0 mm, preferably less than 4.0, more preferably from 3.3 to 0.5 mm and most preferably from 3.0 to 1.0 mm.

6. A pad according to any of the preceding claims wherein the wearer facing surface and/or the garment facing surface of the pad has a coefficient of friction greater than 1.

7. A pad according to any of the preceding claims wherein said pad comprises a liquid permeable and water vapour pervious topsheet, a liquid impermeable, water vapour permeable backsheet and an absorbent core disposed between said topsheet and said backsheet.

8. A pad according to claim 7 wherein said absorbent core has a tensile strength under wet condition of at least 550g/ 25.4mm, preferably from 1000 to 6000 g/25.4mm and more preferably from 1500 to 4000 g/25.4mm.

9. A pad according to any of the preceding claims 7 or 8 wherein said absorbent core typically comprising a fibrous matrix and non granular super absorbent gelling material, preferably fibrous super absorbent gelling material.

10. A pad according to claim 9 wherein the absorbent core comprises from 0.1% to 95%, preferably from 5% to 80% and most preferably from 10% to 55% by weight of the non granular super absorbent gelling material.

11. A pad according to any of the preceding claims 7 to 10 wherein said backsheet is a liquid impermeable, water vapour permeable composite structure comprising a hydrophilic thermoplastic film and a support layer.

**12.** A pad according to claim 11, wherein said thermoplastic film comprises from 5% to 100% by weight of a polymer or mixture of polymers.

**13.** A pad according to claim 12, wherein said polymer is selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, and mixtures thereof.

**14.** A pad according to any of the claims 11 to 13 wherein said hydrophilic thermoplastic film further comprises from 0% to 95%, preferably from 20% to 90% by weight of a plasticiser, typically selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids ($C_8$-$C_{22}$) and their derivatives, and mixtures thereof.

**15.** A pad according to any of the claims 11 to 14 wherein said hydrophilic thermoplastic film further comprises from 0% to 95%, preferably from 2% to 15% by weight of a blend of tackifier resins.

**16.** A pad according to any of the claims 11 to 15 wherein the support layer of the composite structure is selected from the group consisting of wovens, nonwovens and apertured films.

**17.** A pad according to any of the preceding claims 1 to 10 wherein the garment facing surface is provided by a backsheet which is air permeable, typically having an air permeability higher than 50 l/m$^2$/s, preferably higher than 100 l/m$^2$/s, and more preferably higher than 200 l/m$^2$/s.

**18.** A pad according to claim 17 wherein the backsheet comprises at least a first layer made of a resilient, three dimensional web which consists of a liquid impervious polymeric film having apertures, said apertures preferably forming capillaries which are not perpendicular to the plane of the film but are disposed at an angle of less than 90° relative to the plane of the film, and at least a second layer made of a porous web which is a fibrous nonwoven web having a basis weight of less than 100 g/m2 and preferably less than 50.

**19.** A pad according to any of the preceding claims further comprising chitosan material or a mixture thereof preferably at a level of from 0.1 gm$^{-2}$ to 500 gm$^{-2}$, preferably from 0.5 to 200 gm$^{-2}$, more preferably from 3 gm$^{-2}$ to 100 gm$^{-2}$ and most preferably from 4 gm$^{-2}$ to 50 gm$^{-2.}$

**20.** A breathable breast pad having a wearer facing surface and a garment facing surface, wherein at least one of said surfaces has a coefficient of friction greater than 1.

**21.** A pad according to any of the preceding claims 6 or 20 wherein at least one of said surfaces comprises a non-slip material applied thereto, said material being a non-slip latex or hot melt coating typically selected from the group consisting of ethylene vinyl acetate copolymers, polyvinyl acetate, styrenebutadiene, cellulose acetate butyrate, ethyl cellulose, acrylic acid copolymers, elastomers, synthetic rubber hot melt, hot melt polyethylenes, hot melt polyamides and mixtures thereof and more preferably an elastomer containing urethane bonds and/or an alkyl ester of acrylic acid copolymer based material.

Figure 1

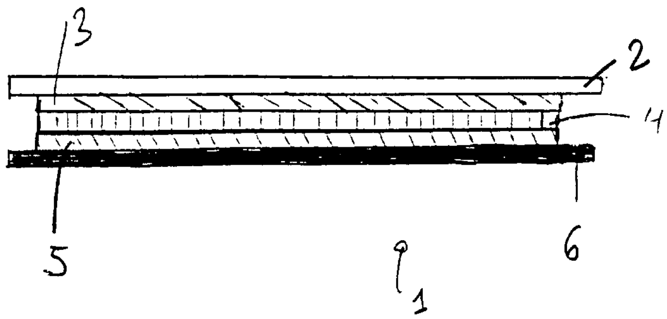


Figure 2

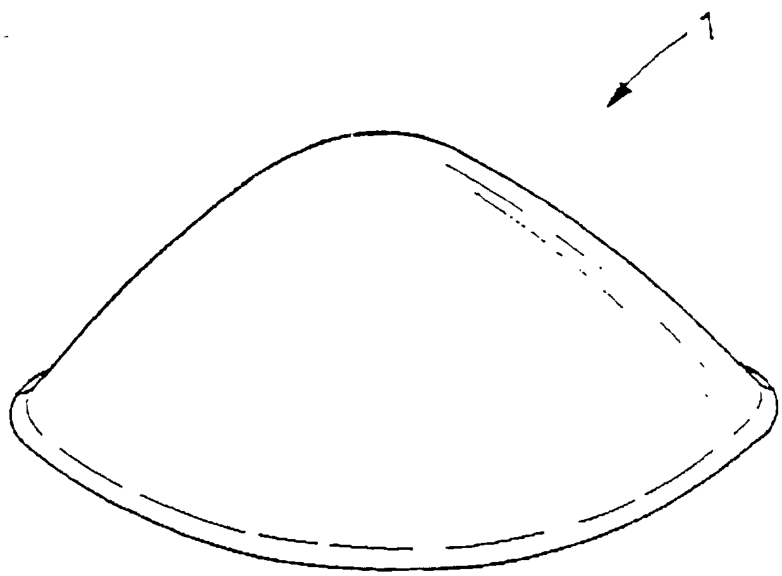

Figure 3

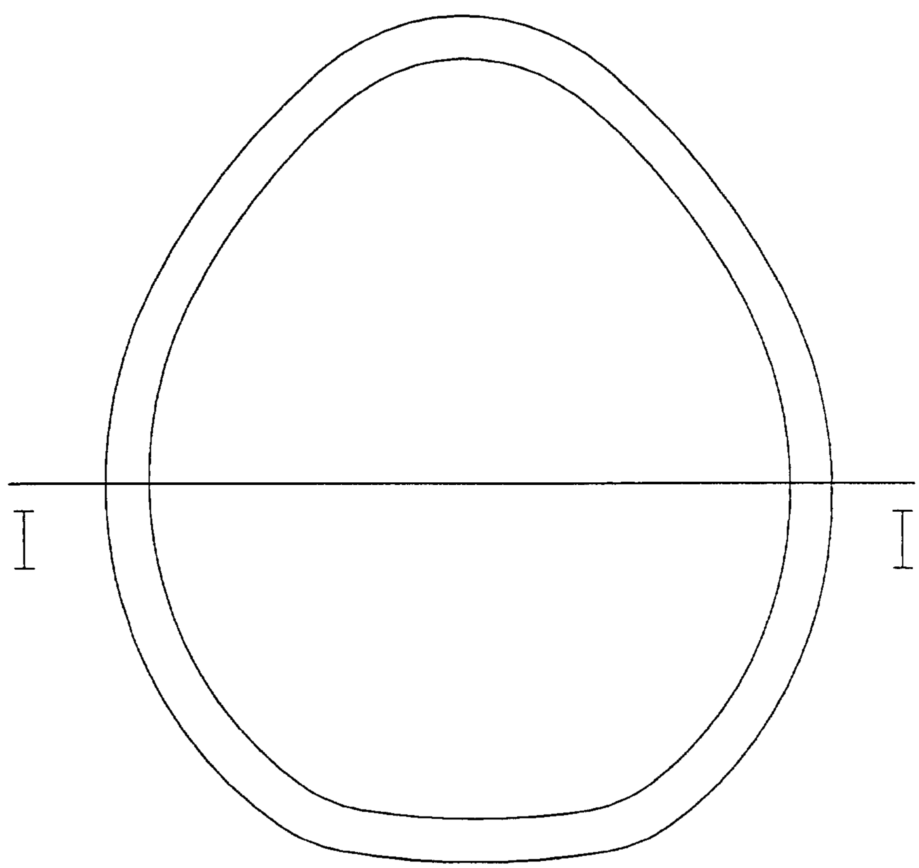

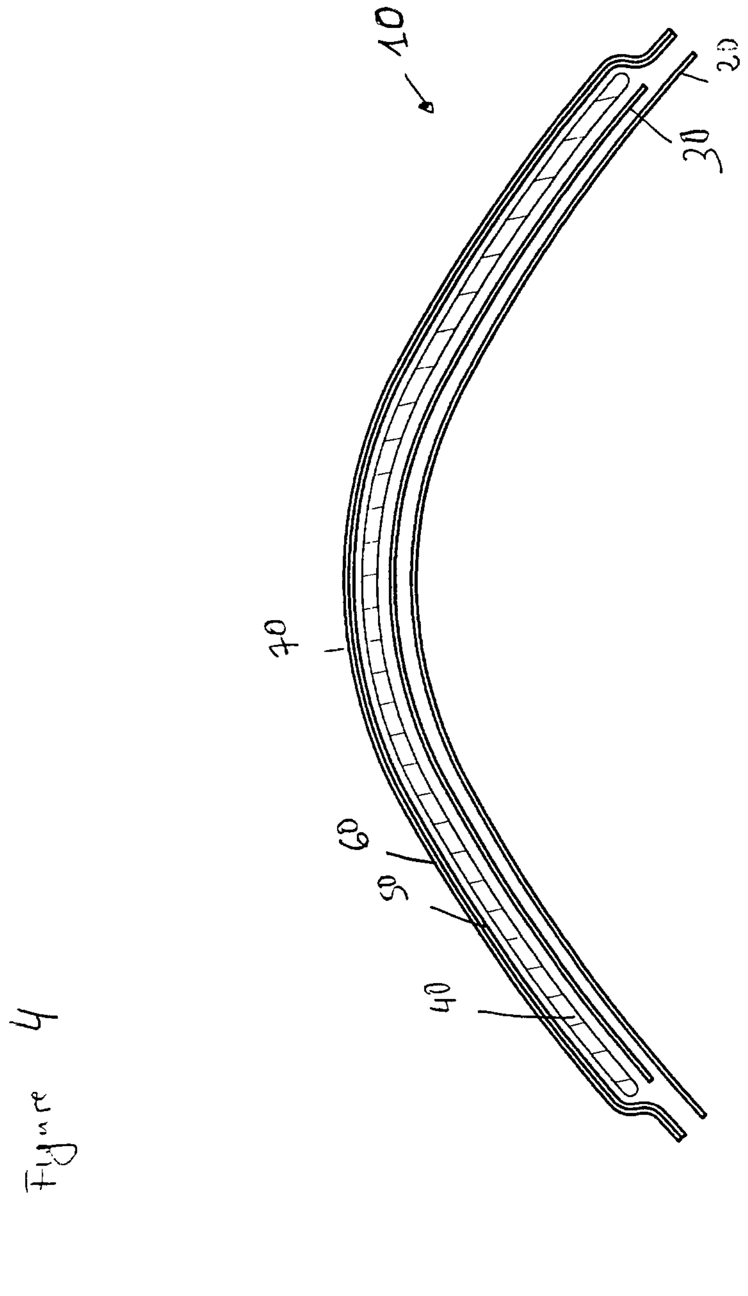
Figure 4
10
20
30
40
50
60
70

European Patent Office

# PARTIAL EUROPEAN SEARCH REPORT

which under Rule 45 of the European Patent Convention shall be considered, for the purposes of subsequent proceedings, as the European search report

Application Number
EP 00 12 2215

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 040 799 A (PROCTER & GAMBLE) 4 October 2000 (2000-10-04)<br>* page 3, line 22 - page 7, line 9 *<br>* page 8, line 19 - line 35 *<br>* page 12, line 9 - page 16, line 11 *<br>--- | 1-3,5,7, 11,12, 16-18 | A61F13/14<br>A61F13/514 |
| X | EP 1 040 800 A (PROCTER & GAMBLE) 4 October 2000 (2000-10-04)<br>* column 3, line 19 - column 11, line 57 *<br>* figures 1-6 *<br>--- | 1-5,7,8, 11,12, 16-18 | |
| X | EP 1 040 806 A (PROCTER & GAMBLE) 4 October 2000 (2000-10-04)<br>* page 3, line 29 - page 8, line 8 *<br>* page 9, line 31 - page 10, line 29 *<br>* figures 1-7 *<br>---<br>-/-- | 1-5,7,8, 11,12, 16-18 | |

TECHNICAL FIELDS SEARCHED (Int.Cl.7)

A61F

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 7 March 2001 | Joly, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# INCOMPLETE SEARCH SHEET C

Application Number
EP 00 12 2215

Claim(s) searched completely:
7,9,11-16,18,19,21

Claim(s) searched incompletely:
1,2,3,4,5,6,17,20

Reason for the limitation of the search:

Present claims 1,2,3,4,5,6,17,20 relate to a breast pad defined by reference to desirable characteristics or properties, namely water vapor permeability, stiffness,fluid absorption capacity, thickness, coefficient of friction, air permeability .

The claims cover all breast pads having these characteristics or properties, whereas the application provides support within the meaning of Article 84 EPC and/or disclosure within the meaning of Article 83 EPC for only a very limited number of such breast pad. In the present case, the claims so lack support, and the application so lacks disclosure, that a meaningful search over the whole of the claimed scope is impossible. Independent of the above reasoning, the claims also lack clarity (Article 84 EPC). An attempt is made to define the breast pad by reference to a result to be achieved. Again, this lack of clarity in the present case is such as to render a meaningful search over the whole of the claimed scope impossible.
Consequently, the search has been carried out for those parts of the claims which appear to be clear, supported and disclosed, namely those parts relating to the breast pads with a topsheet an absorbent core and a breathable backsheet . The backsheet is water vapour permeable and liquid impermeable. The backsheet comprises a film and a nonwoven. The film can be three dimensional and can have capillarities with angle less than 90° to the plane of the film. The backsheet could have anti-slip properties on the garment facing surface. (see Claims 7,9,10,11-16,18,19. Examples 4 and 6. Figures 1,4)

European Patent Office

# PARTIAL EUROPEAN SEARCH REPORT

Application Number
EP 00 12 2215

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 104 396 A (OATLEY JOHN A ET AL) 14 April 1992 (1992-04-14)<br>* column 2, line 1 - line 30 *<br>* column 4, line 48 - column 5, line 6 *<br>* column 5, line 59 - column 7, line 14 *<br>* figures 1-8 *<br>--- | 1-4,6-8, 20 | |
| A | US 4 047 534 A (THOMASCHEFSKY SUSAN N ET AL) 13 September 1977 (1977-09-13)<br>* column 3, line 29 - column 4, line 35 *<br>* figures 1,2 *<br>--- | 6,20 | |
| A | EP 0 324 097 A (KIMBERLY CLARK CO) 19 July 1989 (1989-07-19)<br>* column 2, line 25 - column 6, line 27 *<br>* figures 1-19 *<br>--- | 6,20,21 | TECHNICAL FIELDS SEARCHED (Int.Cl.7) |
| A | WO 99 22685 A (KIMBERLY CLARK CO) 14 May 1999 (1999-05-14)<br>* page 7, line 1 - line 3; figure 4 *<br>--- | 9,10 | |
| A | US 5 432 000 A (RICHARD H YOUNG SR AND AL) 11 July 1995 (1995-07-11)<br>* column 9, line 20 - column 10, line 24 *<br>* column 26, line 1 - line 10 *<br>--- | 19 | |
| A | EP 0 306 262 A (PROCTER & GAMBLE) 8 March 1989 (1989-03-08)<br>* table 2 *<br>--- | 4 | |
| A | EP 0 293 208 A (LION CORP) 30 November 1988 (1988-11-30)<br>* table 3 *<br>--- | 4 | |
| A | WO 99 22059 A (SCA HYGIENE PRODUCTS AB) 6 May 1999 (1999-05-06)<br>* table 2 *<br>----- | 8 | |

EPO FORM 1503 03.82 (P04C10)

## ANNEX TO THE EUROPEAN SEARCH REPORT ON EUROPEAN PATENT APPLICATION NO. EP 00 12 2215

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2001

| Patent document cited in search report | Publication date | Patent family member(s) | Publication date |
|---|---|---|---|
| EP 1040799 A | 04-10-2000 | AU 4191000 A | 23-10-2000 |
| | | WO 0059432 A | 12-10-2000 |
| EP 1040800 A | 04-10-2000 | AU 4191100 A | 23-10-2000 |
| | | WO 0059433 A | 12-10-2000 |
| EP 1040806 A | 04-10-2000 | AU 3317100 A | 23-10-2000 |
| | | WO 0059425 A | 12-10-2000 |
| US 5104396 A | 14-04-1992 | AT 64089 T | 15-06-1991 |
| | | AU 6262686 A | 12-03-1987 |
| | | AU 651564 B | 28-07-1994 |
| | | AU 6575190 A | 17-01-1991 |
| | | CA 1302064 A | 02-06-1992 |
| | | EP 0214867 A | 18-03-1987 |
| | | IE 59193 B | 26-01-1994 |
| | | NZ 217543 A | 26-02-1990 |
| | | ZA 8606898 A | 29-04-1987 |
| US 4047534 A | 13-09-1977 | NONE | |
| EP 0324097 A | 19-07-1989 | US 4834739 A | 30-05-1989 |
| | | AT 80787 T | 15-10-1992 |
| | | AU 2664688 A | 22-06-1989 |
| | | BR 8806700 A | 29-08-1989 |
| | | CA 1323143 A | 19-10-1993 |
| | | DE 3874875 A | 29-10-1992 |
| | | DE 3874875 T | 25-02-1993 |
| | | ES 2035230 T | 16-04-1993 |
| | | JP 2004371 A | 09-01-1990 |
| | | KR 9707088 B | 02-05-1997 |
| | | MX 169373 B | 30-06-1993 |
| | | SG 16993 G | 11-06-1993 |
| | | ZA 8808998 A | 25-10-1989 |
| WO 9922685 A | 14-05-1999 | AU 1106399 A | 24-05-1999 |
| | | DE 19882774 T | 26-10-2000 |
| | | GB 2346902 A | 23-08-2000 |
| US 5432000 A | 11-07-1995 | US 5230959 A | 27-07-1993 |
| | | CA 2106493 A | 23-09-1992 |
| | | EP 0575516 A | 29-12-1993 |
| | | JP 7503284 T | 06-04-1995 |
| | | WO 9216681 A | 01-10-1992 |
| | | US 5498478 A | 12-03-1996 |
| | | AU 5400790 A | 22-10-1990 |



For more details about this annex : see Official Journal of the European Patent Office, No. 12/82

## ANNEX TO THE EUROPEAN SEARCH REPORT ON EUROPEAN PATENT APPLICATION NO. EP 00 12 2215

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2001

| Patent document cited in search report | Publication date | Patent family member(s) | Publication date |
|---|---|---|---|
| US 5432000 A | | CA 2012532 A | 20-09-1990 |
| | | EP 0464143 A | 08-01-1992 |
| | | JP 4504234 T | 30-07-1992 |
| | | US 5516585 A | 14-05-1996 |
| | | WO 9011181 A | 04-10-1990 |
| | | AU 5349790 A | 22-10-1990 |
| | | CA 2012526 A | 20-09-1990 |
| | | EP 0464136 A | 08-01-1992 |
| | | JP 4504233 T | 30-07-1992 |
| | | WO 9011170 A | 04-10-1990 |
| | | AU 5357990 A | 22-10-1990 |
| | | CA 2012524 A | 20-09-1990 |
| | | EP 0464146 A | 08-01-1992 |
| | | JP 4504235 T | 30-07-1992 |
| | | WO 9011171 A | 04-10-1990 |
| EP 0306262 A | 08-03-1989 | US 4865596 A | 12-09-1989 |
| | | AT 92771 T | 15-08-1993 |
| | | CA 1303831 A | 23-06-1992 |
| | | DE 3883113 A | 16-09-1993 |
| | | DE 3883113 T | 02-12-1993 |
| | | ES 2042757 T | 16-12-1993 |
| | | GR 3008729 T | 30-11-1993 |
| | | JP 1153157 A | 15-06-1989 |
| | | JP 2716474 B | 18-02-1998 |
| EP 0293208 A | 30-11-1988 | JP 2626758 B | 02-07-1997 |
| | | JP 63294853 A | 01-12-1988 |
| | | JP 1145061 A | 07-06-1989 |
| | | AT 65411 T | 15-08-1991 |
| | | DE 3863846 D | 29-08-1991 |
| WO 9922059 A | 06-05-1999 | AU 9770598 A | 17-05-1999 |
| | | BR 9813271 A | 22-08-2000 |
| | | CN 1277644 T | 20-12-2000 |
| | | EP 0938601 A | 01-09-1999 |
| | | PL 340215 A | 15-01-2001 |
| | | SE 9703886 A | 25-04-1999 |
| | | US 6163943 A | 26-12-2000 |

EPO FORM P0459

For more details about this annex : see Official Journal of the European Patent Office, No. 12/82